# EUROPEAN PATENT SPECIFICATION

(11) **EP 1 578 969 B1**
(45) Date of publication and mention of the grant of the patent: **07.04.2010**
(21) Application number: 03810343.8
(22) Date of filing: 10.11.2003
(51) Int. Cl.: C12N 15/10, C12N 15/67

(54) **A METHOD FOR OPTIMISING GENE EXPRESSION USING SYNONYMOUS CODON OPTIMISATION**
VERFAHREN ZUR OPTIMIERUNG DER GENEXPRESSION UNTER VERWENDUNG SYNONYMER CODONOPTIMIERUNG
PROCEDE D'OPTIMISATION DE L'EXPRESSION GENIQUE AU MOYEN D'UNE OPTIMISATION DE CODON SYNONYME

(30) Priority: 08.11.2002 US 425163 P
(43) Date of publication of application: 28.09.2005
(73) Proprietor: THE UNIVERSITY OF QUEENSLAND, St Lucia, QLD 4072 (AU)
(72) Inventor: FRAZER, Ian, Hector, ST LUCIA, Queensland 4067 (AU)
(74) Representative: Mallalieu, Catherine Louise
(86) International application number: PCT/AU2003/001487
(87) International publication number: WO 2004/042059

(56) References cited:
- WO-A-00/42190
- WO-A-00/42215
- WO-A-01/66739
- WO-A-97/11086
- WO-A-98/12207
- WO-A-99/02694
- US-A- 5 689 052
- KAWAKAMI, K. ET AL: 'Different lenghts of a polymorphic repeat sequence in the thymidylate synthase gene affect translational efficiency but not its gene expression' CLINICAL CANCER RESEARCH vol. 7, no. 12, 2001, pages 4096 - 4101, XP003007973
- STENSTRöM, C. M. ET AL: 'Influences on translation initiation and early elongation by the messenger RNA region flanking the initiation codon at the 3'side' GENE vol. 288, no. 1-2, 17 April 2002, pages 1 - 8, XP004358891

### SEQUENCE LISTING

<110> The University of Queensland (all states, except U.S.) Frazer, Ian Hector (U.S. only)
<120> A method for optimising gene expression
<130> 12178192/VPA
<140> Unassigned
   <141> 2003-11-10
<150> USSN 60/425,163
   <151> 2002-11-08
<160> 126
<170> PatentIn version 3.2
<210> 1
   <211> 714
   <212> DNA
   <213> Artificial Sequence
<220>
   <223> Humanised GFP
<220>
   <221> CDS
   <222> (1)..(711)
<400> 1
   agc aag ggc gag gaa ctg ttc act ggc gtg gtc cca att ctc gtg gaa      48
   Ser Lys Gly Glu Glu Leu Phe Thr Gly Val Val Pro Ile Leu Val Glu
   1               5                   10                  15
   ctg gat ggc gat gtg aat ggg cac aaa ttt tct gtc agc gga gag ggt      96
   Leu Asp Gly Asp Val Asn Gly His Lys Phe Ser Val Ser Gly Glu Gly
               20                  25                  30
   gaa ggt gat gcc aca tac gga aag ctc acc ctg aaa ttc atc tgc acc     144
   Glu Gly Asp Ala Thr Tyr Gly Lys Leu Thr Leu Lys Phe Ile Cys Thr
           35                  40                  45
   act gga aag ctc cct gtg cca tgg cca aca ctg gtc act acc ttc tct     192
   Thr Gly Lys Leu Pro Val Pro Trp Pro Thr Leu Val Thr Thr Phe Ser
       50                  55                  60
   tat ggc gtg cag tgc ttt tcc aga tac cca gac cat atg aag cag cat     240
   Tyr Gly Val Gln Cys Phe Ser Arg Tyr Pro Asp His Met Lys Gln His
   65                  70                  75                  80
   gac ttt ttc aag agc gcc atg ccc gag ggc tat gtg cag gag aga acc     288
   Asp Phe Phe Lys Ser Ala Met Pro Glu Gly Tyr Val Gln Glu Arg Thr
                   85                  90                  95
   atc ttt ttc aaa gat gac ggg aac tac aag acc cgc gct gaa gtc aag     336
   Ile Phe Phe Lys Asp Asp Gly Asn Tyr Lys Thr Arg Ala Glu Val Lys
               100                 105                 110
   ttc gaa ggt gac acc ctg gtg aat aga atc gag ctg aag ggc att gac     384
   Phe Glu Gly Asp Thr Leu Val Asn Arg Ile Glu Leu Lys Gly Ile Asp
           115                 120                 125
   ttt aag gag gat gga aac att ctc ggc cac aag ctg gaa tac aac tat      432
   Phe Lys Glu Asp Gly Asn Ile Leu Gly His Lys Leu Glu Tyr Asn Tyr
       130                 135                 140
   aac tcc cac aat gtg tac atc atg gcc gac aag caa aag aat ggc atc      480
   Asn Ser His Asn Val Tyr Ile Met Ala Asp Lys Gln Lys Asn Gly Ile
   145                 150                 155                 160
   aag gtc aac ttc aag atc aga cac aac att gag gat gga tcc gtg cag      528
   Lys Val Asn Phe Lys Ile Arg His Asn Ile Glu Asp Gly Ser Val Gln
                   165                 170                 175
   ctg gcc gac cat tat caa cag aac act cca atc ggc gac ggc cct gtg      576
   Leu Ala Asp His Tyr Gln Gln Asn Thr Pro Ile Gly Asp Gly Pro Val
               180                 185                 190
   ctc ctc cca gac aac cat tac ctg tcc acc cag tct gcc ctg tct aaa      624
   Leu Leu Pro Asp Asn His Tyr Leu Ser Thr Gln Ser Ala Leu Ser Lys
           195                 200                 205
   gat ccc aac gaa aag aga gac cac atg gtc ctg ctg gag ttt gtg acc      672
   Asp Pro Asn Glu Lys Arg Asp His Met Val Leu Leu Glu Phe Val Thr
       210                 215                 220
   gct gct ggg atc aca cat ggc atg gac gag ctg tac aag tga              714
   Ala Ala Gly Ile Thr His Gly Met Asp Glu Leu Tyr Lys
   225                 230                 235
<210> 2
   <211> 237
   <212> PRT
   <213> Artificial Sequence
<220>
   <223> Humanised GFP
<400> 2
   Ser Lys Gly Glu Glu Leu Phe Thr Gly Val Val Pro Ile Leu Val Glu
   1               5                   10                  15
   Leu Asp Gly Asp Val Asn Gly His Lys Phe Ser Val Ser Gly Glu Gly
               20                  25                  30
   Glu Gly Asp Ala Thr Tyr Gly Lys Leu Thr Leu Lys Phe Ile Cys Thr
           35                  40                  45
   Thr Gly Lys Leu Pro Val Pro Trp Pro Thr Leu Val Thr Thr Phe Ser
       50                  55                  60
   Tyr Gly Val Gln Cys Phe Ser Arg Tyr Pro Asp His Met Lys Gln His
   65                  70                  75                  80
   Asp Phe Phe Lys Ser Ala Met Pro Glu Gly Tyr Val Gln Glu Arg Thr
                   85                  90                  95
   Ile Phe Phe Lys Asp Asp Gly Asn Tyr Lys Thr Arg Ala Glu Val Lys
               100                 105                 110
   Phe Glu Gly Asp Thr Leu Val Asn Arg Ile Glu Leu Lys Gly Ile Asp
           115                 120                 125
   Phe Lys Glu Asp Gly Asn Ile Leu Gly His Lys Leu Glu Tyr Asn Tyr
       130                 135                 140
   Asn Ser His Asn Val Tyr Ile Met Ala Asp Lys Gln Lys Asn Gly Ile
   145                 150                 155                 160
   Lys Val Asn Phe Lys Ile Arg His Asn Ile Glu Asp Gly Ser Val Gln
                   165                 170                 175
   Leu Ala Asp His Tyr Gln Gln Asn Thr Pro Ile Gly Asp Gly Pro Val
               180                 185                 190
   Leu Leu Pro Asp Asn His Tyr Leu Ser Thr Gln Ser Ala Leu Ser Lys
           195                 200                 205
   Asp Pro Asn Glu Lys Arg Asp His Met Val Leu Leu Glu Phe Val Thr
       210                 215                 220
   Ala Ala Gly Ile Thr His Gly Met Asp Glu Leu Tyr Lys
   225                 230                 235
<210> 3
   <211> 18
   <212> DNA
   <213> Artificial Sequence
<220>
   <223> Ala(GCA)x6
<220>
   <221> CDS
   <222> (1)..(18)
<400> 3
   gca gca gca gca gca gca                                             18
   Ala Ala Ala Ala Ala Ala
   1               5
<210> 4
   <211> 6
   <212> PRT
   <213> Artificial Sequence
<220>
   <223> Ala(GCA)x6
<400> 4
   Ala Ala Ala Ala Ala Ala
   1               5
<210> 5
   <211> 18
   <212> DNA
   <213> Artificial Sequence
<220>
   <223> Ala(GCG)x6
<220>
   <221> CDS
   <222> (1)..(18)
<400> 5
   gcg gcg gcg gcg gcg gcg                                             18
   Ala Ala Ala Ala Ala Ala
   1               5
<210> 6
   <211> 6
   <212> PRT
   <213> Artificial Sequence
<220>
   <223> Ala(GCG)x6
<400> 6
   Ala Ala Ala Ala Ala Ala
   1               5
<210> 7
   <211> 18
   <212> DNA
   <213> Artificial Sequence
<220>
   <223> Ala(GCT)x6
<220>
   <221> CDS
   <222> (1)..(18)
<400> 7
   gct gct gct gct gct gct                                                18
   Ala Ala Ala Ala Ala Ala
   1               5
<210> 8
   <211> 6
   <212> PRT
   <213> Artificial Sequence
<220>
   <223> Ala(GCT)x6
<400> 8
   Ala Ala Ala Ala Ala Ala
   1               5
<210> 9
   <211> 18
   <212> DNA
   <213> Artificial Sequence
<220>
   <223> Ala(GCC)x6
<220>
   <221> CDS
   <222> (1)..(18)
<400> 9
   gcc gcc gcc gcc gcc gcc 18
   Ala Ala Ala Ala Ala Ala
   1 5
<210> 10
   <211> 6
   <212> PRT
   <213> Artificial Sequence
<220>
   <223> Ala(GCC)x6
<400> 10
   Ala Ala Ala Ala Ala Ala
   1 5
<210> 11
   <211> 18
   <212> DNA
   <213> Artificial Sequence
<220>
   <223> Arg(AGA)x6
<220>
   <221> CDS
   <222> (1)..(18)
<400> 11
   aga aga aga aga aga aga 18
   Arg Arg Arg Arg Arg Arg
   1 5
<210> 12
   <211> 6
   <212> PRT
   <213> Artificial Sequence
<220>
   <223> Arg(AGA)x6
<400> 12
   Arg Arg Arg Arg Arg Arg
   1 5
<210> 13
   <211> 18
   <212> DNA
   <213> Artificial Sequence
<220>
   <223> Arg(CGA)x6
<220>
   <221> CDS
   <222> (1)..(18)
<400> 13
   cga cga cga cga cga cga                                         18
   Arg Arg Arg Arg Arg Arg
   1               5
<210> 14
   <211> 6
   <212> PRT
   <213> Artificial Sequence
<220>
   <223> Arg(CGA)x6
<400> 14
   Arg Arg Arg Arg Arg Arg
   1               5
<210> 15
   <211> 18
   <212> DNA
   <213> Artificial Sequence
<220>
   <223> Arg(CGG)x6
<220>
   <221> CDS
   <222> (1)..(18)
<400> 15
   cgg cgg cgg cgg cgg cgg                                         18
   Arg Arg Arg Arg Arg Arg
   1               5
<210> 16
   <211> 6
   <212> PRT
   <213> Artificial Sequence
<220>
   <223> Arg(CGG)x6
<400> 16
   Arg Arg Arg Arg Arg Arg
   1               5
<210> 17
   <211> 18
   <212> DNA
   <213> Artificial Sequence
<220>
   <223> Arg(CGT)x6
<220>
   <221> CDS
   <222> (1)..(18)
<400> 17
   cgt cgt cgt cgt cgt cgt                                                    18
   Arg Arg Arg Arg Arg Arg
   1               5
<210> 18
   <211> 6
   <212> PRT
   <213> Artificial Sequence
<220>
   <223> Arg(CGT)x6
<400> 18
   Arg Arg Arg Arg Arg Arg
   1               5
<210> 19
   <211> 18
   <212> DNA
   <213> Artificial Sequence
<220>
<223> Arg(AGG)x6
<220>
   <221> CDS
   <222> (1)..(18)
<400> 19
   agg agg agg agg agg agg                                                    18
   Arg Arg Arg Arg Arg Arg
   1               5
<210> 20
   <211> 6
   <212> PRT
   <213> Artificial Sequence
<220>
   <223> Arg(AGG)x6
<400> 20
   Arg Arg Arg Arg Arg Arg
   1               5
<210> 21
   <211> 18
   <212> DNA
   <213> Artificial Sequence
<220>
   <223> Arg(CGC)x6
<220>
   <221> CDS
   <222> (1)..(18)
<400> 21
   cgc cgc cgc cgc cgc cgc                                                18
   Arg Arg Arg Arg Arg Arg
   1               5
<210> 22
   <211> 6
   <212> PRT
   <213> Artificial Sequence
<220>
   <223> Arg(CGC)x6
<400> 22
   Arg Arg Arg Arg Arg Arg
   1               5
<210> 23
   <211> 18
   <212> DNA
   <213> Artificial Sequence
<220>
   <223> Asn(AAC)x6
<220>
   <221> CDS
   <222> (1)..(18)
<400> 23
   aac aac aac aac aac aac                                                          18
   Asn Asn Asn Asn Asn Asn
   1               5
<210> 24
   <211> 6
   <212> PRT
   <213> Artificial Sequence
<220>
   <223> Asn(AAC)x6
<400> 24
   Asn Asn Asn Asn Asn Asn
   1               5
<210> 25
   <211> 18
   <212> DNA
   <213> Artificial Sequence
<220>
   <223> Asn(AAT)x6
<220>
   <221> CDS
   <222> (1)..(18)
<400> 25
   aat aat aat aat aat aat                                                          18
   Asn Asn Asn Asn Asn Asn
   1               5
<210> 26
   <211> 6
   <212> PRT
   <213> Artificial Sequence
<220>
   <223> Asn(AAT)x6
<400> 26
   Asn Asn Asn Asn Asn Asn
   1               5
<210> 27
   <211> 18
   <212> DNA
   <213> Artificial Sequence
<220>
   <223> Asp(GAT)x6
<220>
   <221> CDS
   <222> (1)..(18)
<400> 27
   gat gat gat gat gat gat                                            18
   Asp Asp Asp Asp Asp Asp
   1               5
<210> 28
   <211> 6
   <212> PRT
   <213> Artificial Sequence
<220>
   <223> Asp(GAT)x6
<400> 28
   Asp Asp Asp Asp Asp Asp
   1               5
<210> 29
   <211> 18
   <212> DNA
   <213> Artificial Sequence
<220>
   <223> Asp(GAC)x6
<220>
   <221> CDS
   <222> (1)..(18)
<400> 29
   gac gac gac gac gac gac                                            18
   Asp Asp Asp Asp Asp Asp
   1               5
<210> 30
   <211> 6
   <212> PRT
   <213> Artificial Sequence
<220>
   <223> Asp(GAC)x6
<400> 30
   Asp Asp Asp Asp Asp Asp
   1 5
<210> 31
   <211> 18
   <212> DNA
   <213> Artificial Sequence
<220>
   <223> Cys(TGC)x6
<220>
   <221> CDS
   <222> (1)..(18)
<400> 31
   tgc tgc tgc tgc tgc tgc 18
   Cys Cys Cys Cys Cys Cys
   1 5
<210> 32
   <211> 6
   <212> PRT
   <213> Artificial Sequence
<220>
   <223> Cys(TGC)x6
<400> 32
   Cys Cys Cys Cys Cys Cys
   1 5
<210> 33
   <211> 18
   <212> DNA
   <213> Artificial Sequence
<220>
   <223> Cys(TGT)x6
<220>
   <221> CDS
   <222> (1)..(18)
<400> 33
   tgt tgt tgt tgt tgt tgt 18
   Cys Cys Cys Cys Cys Cys
   1 5
<210> 34
   <211> 6
   <212> PRT
   <213> Artificial Sequence
<220>
   <223> Cys(TGT)x6
<400> 34
   Cys Cys Cys Cys Cys Cys
   1               5
<210> 35
   <211> 18
   <212> DNA
   <213> Artificial Sequence
<220>
   <223> Gln(CAA)x6
<220>
   <221> CDS
   <222> (1).. (18)
<400> 35
   caa caa caa caa caa caa                                                18
   Gln Gln Gln Gln Gln Gln
   1               5
<210> 36
   <211> 6
   <212> PRT
   <213> Artificial Sequence
<220>
   <223> Gln(CAA)x6
<400> 36
   Gln Gln Gln Gln Gln Gln
   1               5
<210> 37
   <211> 18
   <212> DNA
   <213> Artificial Sequence
<220>
   <223> Gln(CAG)x6
<220>
   <221> CDS
   <222> (1)..(18)
<400> 37
   cag cag cag cag cag cag                                        18
   Gln Gln Gln Gln Gln Gln
   1               5
<210> 38
   <211> 6
   <212> PRT
   <213> Artificial Sequence
<220>
   <223> Gln (CAG) x6
<400> 38
   Gln Gln Gln Gln Gln Gln
   1               5
<210> 39
   <211> 18
   <212> DNA
   <213> Artificial Sequence
<220>
   <223> Glu(GAA)x6
<220>
   <221> CDS
   <222> (1)..(18)
<400> 39
   gaa gaa gaa gaa gaa gaa                                        18
   Glu Glu Glu Glu Glu Glu
   1               5
<210> 40
   <211> 6
   <212> PRT
   <213> Artificial Sequence
<220>
   <223> Glu(GAA)x6
<400> 40
   Glu Glu Glu Glu Glu Glu
   1               5
<210> 41
   <211> 18
   <212> DNA
   <213> Artificial Sequence
<220>
   <223> Glu(GAG)x6
<220>
   <221> CDS
   <222> (1)..(18)
<400> 41
   gag gag gag gag gag gag 18
<210> 42
   <211> 6
   <212> PRT
   <213> Artificial Sequence
<220>
   <223> Glu(GAG)x6
<400> 42
   Glu Glu Glu Glu Glu Glu
   1 5
<210> 43
   <211> 18
   <212> DNA
   <213> Artificial Sequence
<220>
   <223> Gly(GGA)x6
<220>
   <221> CDS
   <222> (1)..(18)
<400> 43
   gga gga gga gga gga gga 18
   Gly Gly Gly Gly Gly Gly
   1 5
<210> 44
   <211> 6
   <212> PRT
   <213> Artificial Sequence
<220>
   <223> Gly(GGA)x6
<400> 44
   Gly Gly Gly Gly Gly Gly
   1 5
<210> 45
   <211> 18
   <212> DNA
   <213> Artificial Sequence
<220>
   <223> Gly(GGG)x6
<220>
   <221> CDS
   <222> (1)..(18)
<400> 45
   ggg ggg ggg ggg ggg ggg 18
   Gly Gly Gly Gly Gly Gly
   1 5
<210> 46
   <211> 6
   <212> PRT
   <213> Artificial Sequence
<220>
   <223> Gly(GGG)x6
<400> 46
   Gly Gly Gly Gly Gly Gly
   1 5
<210> 47
   <211> 18
   <212> DNA
   <213> Artificial Sequence
<220>
   <223> Gly(GGC)x6
<220>
   <221> CDS
   <222> (1)..(18)
<400> 47
   ggc ggc ggc ggc ggc ggc 18
   Gly Gly Gly Gly Gly Gly
   1 5
<210> 48
   <211> 6
   <212> PRT
   <213> Artificial Sequence
<220>
   <223> Gly(GGC)x6
<400> 48
   Gly Gly Gly Gly Gly Gly
   1               5
<210> 49
   <211> 18
   <212> DNA
   <213> Artificial Sequence
<220>
   <223> Gly(GGT)x6
<220>
   <221> CDS
   <222> (1)..(18)
<400> 49
   ggt ggt ggt ggt ggt ggt                                                   18
   Gly Gly Gly Gly Gly Gly
   1               5
<210> 50
   <211> 6
   <212> PRT
   <213> Artificial Sequence
<220>
   <223> Gly(GGT)x6
<400> 50
   Gly Gly Gly Gly Gly Gly
   1               5
<210> 51
   <211> 18
   <212> DNA
   <213> Artificial Sequence
<220>
   <223> His(CAC)x6
<220>
   <221> CDS
   <222> (1)..(18)
<400> 51
   cac cac cac cac cac cac                                              18
   His His His His His His
   1               5
<210> 52
   <211> 6
   <212> PRT
   <213> Artificial Sequence
<220>
   <223> His(CAC)x6
<400> 52
   His His His His His His
   1               5
<210> 53
   <211> 18
   <212> DNA
   <213> Artificial Sequence
<220>
   <223> His(CAT)x6
<220>
   <221> CDS
   <222> (1)..(18)
<400> 53
   cat cat cat cat cat cat                                              18
   His His His His His His
   1               5
<210> 54
   <211> 6
   <212> PRT
   <213> Artificial Sequence
<220>
   <223> His(CAT)x6
<400> 54
   His His His His His His
   1               5
<210> 55
   <211> 18
   <212> DNA
   <213> Artificial Sequence
<220>
   <223> Ile(ATC)x6
<220>
   <221> CDS
   <222> (1)..(18)
<400> 55
   atc atc atc atc atc atc                                               18
   Ile Ile Ile Ile Ile Ile
   1               5
<210> 56
   <211> 6
   <212> PRT
   <213> Artificial Sequence
<220>
   <223> Ile(ATC)x6
<400> 56
   Ile Ile Ile Ile Ile Ile
   1               5
<210> 57
   <211> 18
   <212> DNA
   <213> Artificial Sequence
<220>
   <223> Ile(ATT)x6
<220>
   <221> CDS
   <222> (1)..(18)
<400> 57
   att att att att att att                                               18
   Ile Ile Ile Ile Ile Ile
   1               5
<210> 58
   <211> 6
   <212> PRT
   <213> Artificial Sequence
<220>
   <223> Ile(ATT)x6
<400> 58
   Ile Ile Ile Ile Ile Ile
   1 5
<210> 59
   <211> 18
   <212> DNA
   <213> Artificial Sequence
<220>
   <223> Ile(ATA)x6
<220>
   <221> CDS
   <222> (1)..(18)
<400> 59
   ata ata ata ata ata ata 18
   Ile Ile Ile Ile Ile Ile
   1 5
<210> 60
   <211> 6
   <212> PRT
   <213> Artificial Sequence
<220>
   <223> Ile(ATA)x6
<400> 60
   Ile Ile Ile Ile Ile Ile
   1 5
<210> 61
   <211> 18
   <212> DNA
   <213> Artificial Sequence
<220>
   <223> Leu(CTC)x6
<220>
   <221> CDS
   <222> (1)..(18)
<400> 61
   ctc ctc ctc ctc ctc ctc                                               18
   Leu Leu Leu Leu Leu Leu
   1               5
<210> 62
   <211> 6
   <212> PRT
   <213> Artificial Sequence
<220>
   <223> Leu(CTC)x6
<400> 62
   Leu Leu Leu Leu Leu Leu
   1               5
<210> 63
   <211> 18
   <212> DNA
   <213> Artificial Sequence
<220>
   <223> Leu(TTG)x6
<220>
   <221> CDS
   <222> (1)..(18)
<400> 63
   ttg ttg ttg ttg ttg ttg                                               18
   Leu Leu Leu Leu Leu Leu
   1               5
<210> 64
   <211> 6
   <212> PRT
   <213> Artificial Sequence
<220>
   <223> Leu(TTG)x6
<400> 64
   Leu Leu Leu Leu Leu Leu
   1               5
<210> 65
   <211> 18
   <212> DNA
   <213> Artificial Sequence
<220>
   <223> Leu(CTA)x6
<220>
   <221> CDS
   <222> (1)..(18)
<400> 65
   cta cta cta cta cta cta                                                   18
   Leu Leu Leu Leu Leu Leu
   1               5
<210> 66
   <211> 6
   <212> PRT
   <213> Artificial Sequence
<220>
   <223> Leu(CTA)x6
<400> 66
   Leu Leu Leu Leu Leu Leu
   1               5
<210> 67
   <211> 18
   <212> DNA
   <213> Artificial Sequence
<220>
   <223> Leu(CTG)x6
<220>
   <221> CDS
   <222> (1)..(18)
<400> 67
   ctg ctg ctg ctg ctg ctg                                                   18
   Leu Leu Leu Leu Leu Leu
   1               5
<210> 68
   <211> 6
   <212> PRT
   <213> Artificial Sequence
<220>
   <223> Leu(CTG)x6
<400> 68
   Leu Leu Leu Leu Leu Leu
   1 5
<210> 69
   <211> 18
   <212> DNA
   <213> Artificial Sequence
<220>
   <223> Leu (TTA) x6
<220>
   <221> CDS
   <222> (1)..(18)
<400> 69
   tta tta tta tta tta tta 18
   Leu Leu Leu Leu Leu Leu
   1 5
<210> 70
   <211> 6
   <212> PRT
   <213> Artificial Sequence
<220>
   <223> Leu(TTA)x6
<400> 70
   Leu Leu Leu Leu Leu Leu
   1 5
<210> 71
   <211> 18
   <212> DNA
   <213> Artificial Sequence
<220>
   <223> Leu(CTT)x6
<220>
   <221> CDS
   <222> (1)..(18)
<400> 71
   ctt ctt ctt ctt ctt ctt 18
   Leu Leu Leu Leu Leu Leu
   1 5
<210> 72
   <211> 6
   <212> PRT
   <213> Artificial Sequence
<220>
   <223> Leu(CTT)x6
<400> 72
   Leu Leu Leu Leu Leu Leu
   1 5
<210> 73
   <211> 18
   <212> DNA
   <213> Artificial Sequence
<220>
   <223> Lys(AAG)x6
<220>
   <221> CDS
   <222> (1)..(18)
<400> 73
   aag aag aag aag aag aag 18
   Lys Lys Lys Lys Lys Lys
   1 5
<210> 74
   <211> 6
   <212> PRT
   <213> Artificial Sequence
<220>
   <223> Lys(AAG)x6
<400> 74
   Lys Lys Lys Lys Lys Lys
   1 5
<210> 75
   <211> 18
   <212> DNA
   <213> Artificial Sequence
<220>
   <223> Lys(AAA)x6
<220>
   <221> CDS
   <222> (1)..(18)
<400> 75
   aaa aaa aaa aaa aaa aaa                                                    18
   Lys Lys Lys Lys Lys Lys
   1               5
<210> 76
   <211> 6
   <212> PRT
   <213> Artificial Sequence
<220>
   <223> Lys(AAA)x6
<400> 76
   Lys Lys Lys Lys Lys Lys
   1               5
<210> 77
   <211> 18
   <212> DNA
   <213> Artificial sequence
<220>
   <223> Phe(TTT)x6
<220>
   <221> CDS
   <222> (1)..(18)
<400> 77
   ttt ttt ttt ttt ttt ttt                                                    18
   Phe Phe Phe Phe Phe Phe
   1               5
<210> 78
   <211> 6
   <212> PRT
   <213> Artificial sequence
<220>
   <223> Phe(TTT)x6
<400> 78
   Phe Phe Phe Phe Phe Phe
   1               5
<210> 79
   <211> 18
   <212> DNA
   <213> Artificial Sequence
<220>
   <223> Phe(TTC)x6
<220>
   <221> CDS
   <222> (1)..(18)
<400> 79
   ttc ttc ttc ttc ttc ttc                                                  18
   Phe Phe Phe Phe Phe Phe
   1               5
<210> 80
   <211> 6
   <212> PRT
   <213> Artificial Sequence
<220>
   <223> Phe(TTC)x6
<400> 80
   Phe Phe Phe Phe Phe Phe
   1               5
<210> 81
   <211> 18
   <212> DNA
   <213> Artificial Sequence
<220>
   <223> Pro(CCC)x6
<220>
   <221> CDS
   <222> (1)..(18)
<400> 81
   ccc ccc ccc ccc ccc ccc                                                  18
   Pro Pro Pro Pro Pro Pro
   1               5
<210> 82
   <211> 6
   <212> PRT
   <213> Artificial Sequence
<220>
   <223> Pro(CCC)x6
<400> 82
   Pro Pro Pro Pro Pro Pro
   1 5
<210> 83
   <211> 18
   <212> DNA
   <213> Artificial Sequence
<220>
   <223> Pro(CCT)x6
<220>
   <221> CDS
   <222> (1)..(18)
<400> 83
   cct cct cct cct cct cct 18
   Pro Pro Pro Pro Pro Pro
   1 5
<210> 84
   <211> 6
   <212> PRT
   <213> Artificial Sequence
<220>
   <223> Pro(CCT)x6
<400> 84
   Pro Pro Pro Pro Pro Pro
   1 5
<210> 85
   <211> 18
   <212> DNA
   <213> Artificial Sequence
<220>
   <223> Pro(CCG)x6
<220>
   <221> CDS
   <222> (1)..(18)
<400> 85
   ccg ccg ccg ccg ccg ccg 18
   Pro Pro Pro Pro Pro Pro
   1 5
<210> 86
   <211> 6
   <212> PRT
   <213> Artificial Sequence
<220>
   <223> Pro (CCG) x6
<400> 86
   Pro Pro Pro Pro Pro Pro
   1 5
<210> 87
   <211> 18
   <212> DNA
   <213> Artificial sequence
<220>
   <223> Pro(CCA)x6
<220>
   <221> CDS
   <222> (1)..(18)
<400> 87
   cca cca cca cca cca cca 18
   Pro Pro Pro Pro Pro Pro
   1 5
<210> 88
   <211> 6
   <212> PRT
   <213> Artificial sequence
<220>
   <223> Pro(CCA)x6
<400> 88
   Pro Pro Pro Pro Pro Pro
   1 5
<210> 89
   <211> 18
   <212> DNA
   <213> Artificial Sequence
<220>
   <223> Ser(AGC)x6
<220>
   <221> CDS
   <222> (1)..(18)
<400> 89
   agc agc agc agc agc agc                                            18
   Ser Ser Ser Ser Ser Ser
   1               5
<210> 90
   <211> 6
   <212> PRT
   <213> Artificial Sequence
<220>
   <223> Ser(AGC)x6
<400> 90
   Ser Ser Ser Ser Ser Ser
   1               5
<210> 91
   <211> 18
   <212> DNA
   <213> Artificial Sequence
<220>
   <223> Ser(TCT)x6
<220>
   <221> CDS
   <222> (1)..(18)
<400> 91
   tct tct tct tct tct tct                                            18
   Ser Ser Ser Ser Ser Ser
   1               5
<210> 92
   <211> 6
   <212> PRT
   <213> Artificial Sequence
<220>
   <223> Ser(TCT)x6
<400> 92
   Ser Ser Ser Ser Ser Ser
   1 5
<210> 93
   <211> 18
   <212> DNA
   <213> Artificial Sequence
<220>
   <223 Ser(AGT)x6
<220>
   <221> CDS
   <222> (1)..(18)
<400> 93
   agt agt agt agt agt agt 18
   Ser Ser Ser Ser Ser Ser
   1 5
<210> 94
   <211> 6
   <212> PRT
   <213> Artificial Sequence
<220>
   <223> Ser(AGT)x6
<400> 94
   Ser Ser Ser Ser Ser Ser
   1 5
<210> 95
   <211> 18
   <212> DNA
   <213> Artificial Sequence
<220>
   <223> Ser(TCG)x6
<220>
   <221> CDS
   <222> (1)..(18)
<400> 95
   tcg tcg tcg tcg tcg tcg 18
   Ser Ser Ser Ser Ser Ser
   1 5
<210> 96
   <211> 6
   <212> PRT
   <213> Artificial Sequence
<220>
   <223> Ser(TCG)x6
<400> 96
   Ser Ser Ser Ser Ser Ser
   1               5
<210> 97
   <211> 18
   <212> DNA
   <213> Artificial Sequence
<220>
   <223> Ser(TCA)x6
<220>
   <221> CDS
   <222> (1)..(18)
<400> 97
   tca tca tca tca tca tca                                        18
   Ser Ser Ser Ser Ser Ser
   1               5
<210> 98
   <211> 6
   <212> PRT
   <213> Artificial Sequence
<220>
   <223> Ser(TCA)x6
<400> 98
   Ser Ser Ser Ser Ser Ser
   1               5
<210> 99
   <211> 18
   <212> DNA
   <213> Artificial Sequence
<220>
   <223> Ser(TCC)x6
<220>
   <221> CDS
   <222> (1)..(18)
<400> 99
   tcc tcc tcc tcc tcc tcc                                                18
   Ser Ser Ser Ser Ser Ser
   1               5
<210> 100
   <211> 6
   <212> PRT
   <213> Artificial Sequence
<220>
   <223> Ser(TCC)x6
<400> 100
   Ser Ser Ser Ser Ser Ser
   1               5
<210> 101
   <211> 18
   <212> DNA
   <213> Artificial Sequence
<220>
   <223> Thr(ACA)x6
<220>
   <221> CDS
   <222> (1)..(18)
<400> 101
   aca aca aca aca aca aca                                                18
   Thr Thr Thr Thr Thr Thr
   1               5
<210> 102
   <211> 6
   <212> PRT
   <213> Artificial Sequence
<220>
   <223> Thr(ACA)x6
<400> 102
   Thr Thr Thr Thr Thr Thr
   1               5
<210> 103
   <211> 18
   <212> DNA
   <213> Artificial Sequence
<220>
   <223> Thr(ACG)x6
<220>
   <221> CDS
   <222> (1)..(18)
<400> 103
   acg acg acg acg acg acg                                                   18
   Thr Thr Thr Thr Thr Thr
   1               5
<210> 104
   <211> 6
   <212> PRT
   <213> Artificial Sequence
<220>
   <223> Thr(ACG)x6
<400> 104
   Thr Thr Thr Thr Thr Thr
   1               5
<210> 105
   <211> 18
   <212> DNA
   <213> Artificial Sequence
<220>
   <223> Thr(ACT)x6
<220>
   <221> CDS
   <222> (1)..(18)
<400> 105
   act act act act act act                                                   18
   Thr Thr Thr Thr Thr Thr
   1               5
<210> 106
   <211> 6
   <212> PRT
   <213> Artificial Sequence
<220>
   <223> Thr(ACT)x6
<400> 106
   Thr Thr Thr Thr Thr Thr
   1 5
<210> 107
   <211> 18
   <212> DNA
   <213> Artificial Sequence
<220>
   <223> Thr(ACC)x6
<220>
   <221> CDS
   <222> (1)..(18)
<400> 107
   acc acc acc acc acc acc 18
   Thr Thr Thr Thr Thr Thr
   1 5
<210> 108
   <211> 6
   <212> PRT
   <213> Artificial Sequence
<220>
   <223> Thr(ACC)x6
<400> 108
   Thr Thr Thr Thr Thr Thr
   1 5
<210> 109
   <211> 18
   <212> DNA
   <213> Artificial Sequence
<220>
   <223> Tyr(TAC)x6
<220>
   <221> CDS
   <222> (1)..(18)
<400> 109
   tac tac tac tac tac tac 18
   Tyr Tyr Tyr Tyr Tyr Tyr
   1 5
<210> 110
   <211> 6
   <212> PRT
   <213> Artificial Sequence
<220>
   <223> Tyr(TAC)x6
<400> 110
   Tyr Tyr Tyr Tyr Tyr Tyr
   1               5
<210> 111
   <211> 18
   <212> DNA
   <213> Artificial Sequence
<220>
   <223> Tyr(TAT)x6
<220>
   <221> CDS
   <222> (1)..(18)
<400> 111
   tat tat tat tat tat tat                                                   18
   Tyr Tyr Tyr Tyr Tyr Tyr
   1               5
<210> 112
   <211> 6
   <212> PRT
   <213> Artificial Sequence
<220>
   <223> Tyr(TAT)x6
<400> 112
   Tyr Tyr Tyr Tyr Tyr Tyr
   1               5
<210> 113
   <211> 18
   <212> DNA
   <213> Artificial Sequence
<220>
   <223> Val(GTG)x6
<220>
   <221> CDS
   <222> (1)..(18)
<400> 113
   gtg gtg gtg gtg gtg gtg                                                          18
   Val Val Val Val Val Val
   1               5
<210> 114
   <211> 6
   <212> PRT
   <213> Artificial Sequence
<220>
   <223> Val(GTG)x6
<400> 114
   Val Val Val Val Val Val
   1               5
<210> 115
   <211> 18
   <212> DNA
   <213> Artificial Sequence
<220>
   <223> Val(GTT)x6
<220>
   <221> CDS
   <222> (1)..(18)
<400> 115
   gtt gtt gtt gtt gtt gtt                                                          18
   Val Val Val Val Val Val
   1               5
<210> 116
   <211> 6
   <212> PRT
   <213> Artificial Sequence
<220>
   <223> Val(GTT)x6
<400> 116
   Val Val Val Val Val Val
   1               5
<210> 117
   <211> 18
   <212> DNA
   <213> Artificial Sequence
<220>
   <223> Val(GTC)x6
<220>
   <221> CDS
   <222> (1)..(18)
<400> 117
   gtc gtc gtc gtc gtc gtc                                    18
   Val Val Val Val Val Val
   1               5
<210> 118
   <211> 6
   <212> PRT
   <213> Artificial Sequence
<220>
   <223> Val(GTC)x6
<400> 118
   Val Val Val Val Val Val
   1               5
<210> 119
   <211> 18
   <212> DNA
   <213> Artificial Sequence
<220>
   <223> Val(GTA)x6
<220>
   <221> CDS
   <222> (1) .. (18)
<400> 119
   gta gta gta gta gta gta                                    18
   Val Val Val Val Val Val
   1               5
<210> 120
   <211> 6
   <212> PRT
   <213> Artificial Sequence
<220>
   <223> Val(GTA)×6
<400> 120
   Val Val Val Val Val Val
   1 5
<210> 121
   <211> 2583
   <212> DNA
   <213> Mouse
<220>
   <221> CDS
   <222> (1)..(2166)
<400> 121
   gaa ctt cgg gac gag caa act ccg ggc cac agg aag aac cca tcg aac       48
   Glu Leu Arg Asp Glu Gln Thr Pro Gly His Arg Lys Asn Pro Ser Asn
   1               5                   10                  15
   caa agc agc tta gaa tct gac tcc aat tac ccc tcc att tcc act tcc       96
   Gln Ser Ser Leu Glu Ser Asp Ser Asn Tyr Pro Ser Ile Ser Thr Ser
               20                  25                  30
   gaa atc gga gac act gag gat gcc ctt cag cag gtg gag gag att ggc      144
   Glu Ile Gly Asp Thr Glu Asp Ala Leu Gln Gln Val Glu Glu Ile Gly
           35                  40                  45
   ata gag aag gca gcc atg gac atg acc gtc ttc ctg aag ctg cag aag      192
   Ile Glu Lys Ala Ala Met Asp Met Thr Val Phe Leu Lys Leu Gln Lys
       50                  55                  60
   aga gtg cgc gaa ctt gag cag gag agg aag aag ctg cag gcg cag cta      240
   Arg Val Arg Glu Leu Glu Gln Glu Arg Lys Lys Leu Gln Ala Gln Leu
   65                  70                  75                  80
   gaa aag gga cag cag gac agc aag aaa ggg cag gta gaa caa cag aac      288
   Glu Lys Gly Gln Gln Asp Ser Lys Lys Gly Gln Val Glu Gln Gln Asn
                   85                  90                  95
   aat ggc tta gat gtg gac cag gac gca gat ata gcc tac aat agt ctg      336
   Asn Gly Leu Asp Val Asp Gln Asp Ala Asp Ile Ala Tyr Asn Ser Leu
               100                 105                 110
   aag aga cag gag ctt gag tca gag aac aag aag ctg aag aat gac ctg      384
   Lys Arg Gln Glu Leu Glu Ser Glu Asn Lys Lys Leu Lys Asn Asp Leu
           115                 120                 125
   aat gag ctg agg aac ggt gtc gct gac caa gcc atg cag gat aac tcc      432
   Asn Glu Leu Arg Asn Gly Val Ala Asp Gln Ala Met Gln Asp Asn Ser
       130                 135                 140
   acc cac agc tcc cca gac agc tac agc ctc cta ctg aac cag ctc aag      480
   Thr His Ser Ser Pro Asp Ser Tyr Ser Leu Leu Leu Asn Gln Leu Lys
   145                 150                 155                 160
   ctg gcc aat gag gag ctc gag gtc cgc aaa gag gag gcg ctg atc ctc      528
   Leu Ala Asn Glu Glu Leu Glu Val Arg Lys Glu Glu Ala Leu Ile Leu
                   165                 170                 175
   agg acc cag atc atg aat gcc gac cag cgc cgc ctg tct ggc aag aac      576
   Arg Thr Gln Ile Met Asn Ala Asp Gln Arg Arg Leu Ser Gly Lys Asn
               180                 185                 190
   atg gag ccg aac atc aat gcc aga aca agt tgg ccc aac agt gag aag      624
   Met Glu Pro Asn Ile Asn Ala Arg Thr Ser Trp Pro Asn Ser Glu Lys
           195                 200                 205
   cac gtg gac cag gaa gac gcc att gag gcc tat cac ggg gtc tgc cag      672
   His Val Asp Gln Glu Asp Ala Ile Glu Ala Tyr His Gly Val Cys Gln
       210                 215                 220
   aca aac agg ttg ctg gag gcc cag ctg cag gcc cag agc ctg gag cat      720
   Thr Asn Arg Leu Leu Glu Ala Gln Leu Gln Ala Gln Ser Leu Glu His
   225                 230                 235                 240
   gag gag gag gtg gaa cat ctc aag gcc cag gtg gaa gcc ctg aaa gag      768
   Glu Glu Glu Val Glu His Leu Lys Ala Gln Val Glu Ala Leu Lys Glu
                   245                 250                 255
   gag atg gac aaa cag cag cag acc ttc tgc cag acc ctg ctg ctc tcc      816
   Glu Met Asp Lys Gln Gln Gln Thr Phe Cys Gln Thr Leu Leu Leu Ser
               260                 265                 270
   cca gag gcc cag gta gaa ttt ggt gtc cag cag gag ata tcc cgg ctg      864
   Pro Glu Ala Gln Val Glu Phe Gly Val Gln Gln Glu Ile Ser Arg Leu
           275                 280                 285
   acc aat gag aac ctg gat ttt aag gaa ttg gtg gaa aag ctg gag aag      912
   Thr Asn Glu Asn Leu Asp Phe Lys Glu Leu Val Glu Lys Leu Glu Lys
       290                 295                 300
   aat gag agg aag ctg aag aag cag ctg aag att tac atg aag aag gtc      960
   Asn Glu Arg Lys Leu Lys Lys Gln Leu Lys Ile Tyr Met Lys Lys Val
   305                 310                 315                 320
   cag gac tta gaa gct gcc cag gcg ttg gca cag agt gac agg agg cac     1008
   Gln Asp Leu Glu Ala Ala Gln Ala Leu Ala Gln Ser Asp Arg Arg His
                   325                 330                 335
   cat gaa ctc aca aga cag gtc aca gtc caa cga aaa gag aag gac ttc     1056
   His Glu Leu Thr Arg Gln Val Thr Val Gln Arg Lys Glu Lys Asp Phe
               340                 345                 350
   caa ggc atg ctg gag tac cac aaa gag gtc gaa gcc ctc ctc atc cgg     1104
   Gln Gly Met Leu Glu Tyr His Lys Glu Val Glu Ala Leu Leu Ile Arg
           355                 360                 365
   aac ctg gtg aca gac ctg aag cct cag atg ctg ctg ggc acc gtg ccc     1152
   Asn Leu Val Thr Asp Leu Lys Pro Gln Met Leu Leu Gly Thr Val Pro
       370                 375                 380
   tgt ctg cct gca tac ata ctc tat atg tgc atc agg cac gcg gat tac     1200
   Cys Leu Pro Ala Tyr Ile Leu Tyr Met Cys Ile Arg His Ala Asp Tyr
   385                 390                 395                 400
   acc aac gat gac ctc aag gtg cac tcg ttg ctg agc tcc acc atc aac     1248
   Thr Asn Asp Asp Leu Lys Val His Ser Leu Leu Ser Ser Thr Ile Asn
                   405                 410                 415
   ggc att aag aaa gtc ctc aag aag cac aat gac gac ttt gag atg acg     1296
   Gly Ile Lys Lys Val Leu Lys Lys His Asn Asp Asp Phe Glu Met Thr
               420                 425                 430
   tca ttc tgg tta tcc aac acc tgc cgc ttc ctt cac tgt ctg aag caa     1344
   Ser Phe Trp Leu Ser Asn Thr Cys Arg Phe Leu His Cys Leu Lys Gln
           435                 440                 445
   tac agt ggt gat gag ggt ttc atg aca cag aac atc gcg aag cag aat      1392
   Tyr Ser Gly Asp Glu Gly Phe Met Thr Gln Asn Ile Ala Lys Gln Asn
       450                 455                 460
   gag cac tgt ctc aag aac ttt gac ctc act gaa tac cgc cag gta cta      1440
   Glu His Cys Leu Lys Asn Phe Asp Leu Thr Glu Tyr Arg Gln Val Leu
   465                 470                 475                 480
   agc gac ctt tcc att cag atc tat cag cag ctc att aaa atg ccc gag      1488
   Ser Asp Leu Ser Ile Gln Ile Tyr Gln Gln Leu Ile Lys Met Pro Glu
                   485                 490                 495
   ggc ttg cta cag cct atg ata gtt tct gcc atg ttg gaa aat gag agt      1536
   Gly Leu Leu Gln Pro Met Ile Val Ser Ala Met Leu Glu Asn Glu Ser
               500                 505                 510
   atc cag ggg ctg tct ggt gtg aga cca act ggt tac cgg aag cgc tcc      1584
   Ile Gln Gly Leu Ser Gly Val Arg Pro Thr Gly Tyr Arg Lys Arg Ser
           515                 520                 525
   tcc agc atg gtg gat gga gag aat tct ttc cat aca gtc ctg tgt gac      1632
   Ser Ser Met Val Asp Gly Glu Asn Ser Phe His Thr Val Leu Cys Asp
       530                 535                 540
   cag ggc ctg gac ccc gag att atc ctg cag gtg ttc aaa cag ctc ttc      1680
   Gln Gly Leu Asp Pro Glu Ile Ile Leu Gln Val Phe Lys Gln Leu Phe
   545                 550                 555                 560
   tac atg atc aat gct gtg act ctt aac aac cta ctc ctg cgg aaa gac      1728
   Tyr Met Ile Asn Ala Val Thr Leu Asn Asn Leu Leu Leu Arg Lys Asp
                   565                 570                 575
   gcc tgc tcc tgg agc aca ggc atg caa ctc agg tac aac ata agt caa      1776
   Ala Cys Ser Trp Ser Thr Gly Met Gln Leu Arg Tyr Asn Ile Ser Gln
               580                 585                 590
   ctg gaa gag tgg ctt cgg ggc aaa aac ctt cac cag agt gga gca gtt      1824
   Leu Glu Glu Trp Leu Arg Gly Lys Asn Leu His Gln Ser Gly Ala Val
           595                 600                 605
   cag acc atg gag ccc ctg atc cag gca gcc cag ctc ctc cag ctg aag      1872
   Gln Thr Met Glu Pro Leu Ile Gln Ala Ala Gln Leu Leu Gln Leu Lys
       610                 615                 620
   aag aaa acc cac gag gat gct gag gcc atc tgc tct ctg tgc acc tcc      1920
   Lys Lys Thr His Glu Asp Ala Glu Ala Ile Cys Ser Leu Cys Thr Ser
   625                 630                 635                 640
   ctc agc acc cag cag att gtc aaa att tta aac ctc tac act ccc ttg      1968
   Leu Ser Thr Gln Gln Ile Val Lys Ile Leu Asn Leu Tyr Thr Pro Leu
                   645                 650                 655
   aat gaa ttt gag gaa cgg gtc aca gtg tcc ttc atc aga aca atc cag      2016
   Asn Glu Phe Glu Glu Arg Val Thr Val Ser Phe Ile Arg Thr Ile Gln
               660                 665                 670
   gct cag cta caa gag agg aat gac cct cag cag ctc ctg ctg gac tcc      2064
   Ala Gln Leu Gln Glu Arg Asn Asp Pro Gln Gln Leu Leu Leu Asp Ser
           675                 680                 685
   aag cac gtg ttc cca gtt ctg ttt cca tat aac cca tct gct ctg acc      2112
   Lys His Val Phe Pro Val Leu Phe Pro Tyr Asn Pro Ser Ala Leu Thr
       690                 695                 700
   atg gac tcg atc cac atc ccg gcc tgt ctc aac ctg gag ttt ctc aat      2160
   Met Asp Ser Ile His Ile Pro Ala Cys Leu Asn Leu Glu Phe Leu Asn
   705                 710                 715                 720
   gaa gtc tgaggatgcg tgtttccgag gcgagcgaga aggaagcatg tgctgtcagc       2216
   Glu Val
   cgagagaatg ctaggtgtgt taaatattcc agcgtagatc aaaccatgtt agagactggc    2276
   gggacgacag aactaaacag cggggtgcac agttgtcgcc aatgctgctc agaaaacacc    2336
   cggaagtgga tttgttaaag ctgtgctttc aggttaaacc aagacacgtc agaacgaaca    2396
   gccactctgc agctccagtc gccatataaa aatgccagtt ctacagagtg gaagtgccta    2456
   gctttgatct ttgtatatat cttgagaatg ttcaaactga gataatatta aaaacacatg    2516
   acgtaaattg cctttgtggg tctttcaaga aatgatggga ctaataacca taagattgac    2576
   aggaatt                                                              2583
<210> 122
   <211> 722
   <212> PRT
   <213> Mouse
<400> 122
   Glu Leu Arg Asp Glu Gln Thr Pro Gly His Arg Lys Asn Pro Ser Asn
   1               5                   10                  15
   Gln Ser Ser Leu Glu Ser Asp Ser Asn Tyr Pro Ser Ile Ser Thr Ser
               20                  25                  30
   Glu Ile Gly Asp Thr Glu Asp Ala Leu Gln Gln Val Glu Glu Ile Gly
           35                  40                  45
   Ile Glu Lys Ala Ala Met Asp Met Thr Val Phe Leu Lys Leu Gln Lys
       50                  55                  60
   Arg Val Arg Glu Leu Glu Gln Glu Arg Lys Lys Leu Gln Ala Gln Leu
   65                  70                  75                  80
   Glu Lys Gly Gln Gln Asp Ser Lys Lys Gly Gln Val Glu Gln Gln Asn
                   85                  90                  95
   Asn Gly Leu Asp Val Asp Gln Asp Ala Asp Ile Ala Tyr Asn Ser Leu
               100                 105                 110
   Lys Arg Gln Glu Leu Glu Ser Glu Asn Lys Lys Leu Lys Asn Asp Leu
           115                 120                 125
   Asn Glu Leu Arg Asn Gly Val Ala Asp Gln Ala Met Gln Asp Asn Ser
       130                 135                 140
   Thr His Ser Ser Pro Asp Ser Tyr Ser Leu Leu Leu Asn Gln Leu Lys
   145                 150                 155                 160
   Leu Ala Asn Glu Glu Leu Glu Val Arg Lys Glu Glu Ala Leu Ile Leu
                   165                 170                 175
   Arg Thr Gln Ile Met Asn Ala Asp Gln Arg Arg Leu Ser Gly Lys Asn
               180                 185                 190
   Met Glu Pro Asn Ile Asn Ala Arg Thr Ser Trp Pro Asn Ser Glu Lys
           195                 200                 205
   His Val Asp Gln Glu Asp Ala Ile Glu Ala Tyr His Gly Val Cys Gln
       210                 215                 220
   Thr Asn Arg Leu Leu Glu Ala Gln Leu Gln Ala Gln Ser Leu Glu His
   225                 230                 235                 240
   Glu Glu Glu Val Glu His Leu Lys Ala Gln Val Glu Ala Leu Lys Glu
                   245                 250                 255
   Glu Met Asp Lys Gln Gln Gln Thr Phe Cys Gln Thr Leu Leu Leu Ser
               260                 265                 270
   Pro Glu Ala Gln Val Glu Phe Gly Val Gln Gln Glu Ile Ser Arg Leu
           275                 280                 285
   Thr Asn Glu Asn Leu Asp Phe Lys Glu Leu Val Glu Lys Leu Glu Lys
       290                 295                 300
   Asn Glu Arg Lys Leu Lys Lys Gln Leu Lys Ile Tyr Met Lys Lys Val
   305                 310                 315                 320
   Gln Asp Leu Glu Ala Ala Gln Ala Leu Ala Gln Ser Asp Arg Arg His
                   325                 330                 335
   His Glu Leu Thr Arg Gln Val Thr Val Gln Arg Lys Glu Lys Asp Phe
               340                 345                 350
   Gln Gly Met Leu Glu Tyr His Lys Glu Val Glu Ala Leu Leu Ile Arg
           355                 360                 365
   Asn Leu Val Thr Asp Leu Lys Pro Gln Met Leu Leu Gly Thr Val Pro
   370 375 380
   Cys Leu Pro Ala Tyr Ile Leu Tyr Met Cys Ile Arg His Ala Asp Tyr
   385 390 395 400
   Thr Asn Asp Asp Leu Lys Val His Ser Leu Leu Ser Ser Thr Ile Asn
   405 410 415
   Gly Ile Lys Lys Val Leu Lys Lys His Asn Asp Asp Phe Glu Met Thr
   420 425 430
   Ser Phe Trp Leu Ser Asn Thr Cys Arg Phe Leu His Cys Leu Lys Gln
   435 440 445
   Tyr Ser Gly Asp Glu Gly Phe Met Thr Gln Asn Ile Ala Lys Gln Asn
   450 455 460
   Glu His Cys Leu Lys Asn Phe Asp Leu Thr Glu Tyr Arg Gln Val Leu
   465 470 475 480
   Ser Asp Leu Ser Ile Gln Ile Tyr Gln Gln Leu Ile Lys Met Pro Glu
   485 490 495
   Gly Leu Leu Gln Pro Met Ile Val Ser Ala Met Leu Glu Asn Glu Ser
   500 505 510
   Ile Gln Gly Leu Ser Gly Val Arg Pro Thr Gly Tyr Arg Lys Arg Ser
   515 520 525
   Ser Ser Met Val Asp Gly Glu Asn Ser Phe His Thr Val Leu Cys Asp
   530 535 540
   Gln Gly Leu Asp Pro Glu Ile Ile Leu Gln Val Phe Lys Gln Leu Phe
   545 550 555 560
   Tyr Met Ile Asn Ala Val Thr Leu Asn Asn Leu Leu Leu Arg Lys Asp
   565 570 575
   Ala Cys Ser Trp Ser Thr Gly Met Gln Leu Arg Tyr Asn Ile Ser Gln
   580 585 590
   Leu Glu Glu Trp Leu Arg Gly Lys Asn Leu His Gln Ser Gly Ala Val
   595 600 605
   Gln Thr Met Glu Pro Leu Ile Gln Ala Ala Gln Leu Leu Gln Leu Lys
       610                 615                 620
   Lys Lys Thr His Glu Asp Ala Glu Ala Ile Cys Ser Leu Cys Thr Ser
   625                 630                 635                 640
   Leu Ser Thr Gln Gln Ile Val Lys Ile Leu Asn Leu Tyr Thr Pro Leu
                   645                 650                 655
   Asn Glu Phe Glu Glu Arg Val Thr Val Ser Phe Ile Arg Thr Ile Gln
               660                 665                 670
   Ala Gln Leu Gln Glu Arg Asn Asp Pro Gln Gln Leu Leu Leu Asp Ser
           675                 680                 685
   Lys His Val Phe Pro Val Leu Phe Pro Tyr Asn Pro Ser Ala Leu Thr
       690                 695                 700
   Met Asp Ser Ile His Ile Pro Ala Cys Leu Asn Leu Glu Phe Leu Asn
   705                 710                 715                 720
   Glu Val
<210> 123
   <211> 549
   <212> DNA
   <213> Artificial Sequence
<220>
   <223> BAR gene
<220>
   <221> CDS
   <222> (1)..(549)
<400> 123
   agc cca gaa cga cgc ccg gcc gac atc cgc cgt gcc acc gag gcg gac        48
   Ser Pro Glu Arg Arg Pro Ala Asp Ile Arg Arg Ala Thr Glu Ala Asp
   1               5                   10                  15
   atg ccg gcg gtc tgc acc atc gtc aac cac tac atc gag aca agc acg        96
   Met Pro Ala Val Cys Thr Ile Val Asn His Tyr Ile Glu Thr Ser Thr
               20                  25                  30
   gtc aac ttc cgt acc gag ccg cag gaa ccg cag gag tgg acg gac gac       144
   Val Asn Phe Arg Thr Glu Pro Gln Glu Pro Gln Glu Trp Thr Asp Asp
           35                  40                  45
   ctc gtc cgt ctg cgg gag cgc tat ccc tgg ctc gtc gcc gag gtg gac       192
   Leu Val Arg Leu Arg Glu Arg Tyr Pro Trp Leu Val Ala Glu Val Asp
       50                  55                  60
   ggc gag gtc gcc ggc atc gcc tac gcg ggc ccc tgg aag gca cgc aac       240
   Gly Glu Val Ala Gly Ile Ala Tyr Ala Gly Pro Trp Lys Ala Arg Asn
   65                  70                  75                  80
   gcc tac gac tgg acg gcc gag tcg acc gtg tac gtc tcc ccc cgc cac       288
   Ala Tyr Asp Trp Thr Ala Glu Ser Thr Val Tyr Val Ser Pro Arg His
                   85                  90                  95
   cag cgg acg gga ctg ggc tcc acg ctc tac acc cac ctg ctg aag tcc       336
   Gln Arg Thr Gly Leu Gly Ser Thr Leu Tyr Thr His Leu Leu Lys Ser
               100                 105                 110
   ctg gag gca cag ggc ttc aag agc gtg gtc gct gtc atc ggg ctg ccc       384
   Leu Glu Ala Gln Gly Phe Lys Ser Val Val Ala Val Ile Gly Leu Pro
           115                 120                 125
   aac gac ccg agc gtg cgc atg cac gag gcg ctc gga tat gcc ccc cgc       432
   Asn Asp Pro Ser Val Arg Met His Glu Ala Leu Gly Tyr Ala Pro Arg
       130                 135                 140
   ggc atg ctg cgg gcg gcc ggc ttc aag cac ggg aac tgg cat gac gtg       480
   Gly Met Leu Arg Ala Ala Gly Phe Lys His Gly Asn Trp His Asp Val
   145                 150                 155                 160
   ggt ttc tgg cag ctg gac ttc agc ctg ccg gtg ccg ccc cgt ccg gtc       528
   Gly Phe Trp Gln Leu Asp Phe Ser Leu Pro Val Pro Pro Arg Pro Val
                   165                 170                 175
   ctg ccc gtc acc gaa atc tga                                           549
   Leu Pro Val Thr Glu Ile
               180
<210> 124
   <211> 182
   <212> PRT
   <213> Artificial Sequence
<220>
   <223> BAR gene
<400> 124
   Ser Pro Glu Arg Arg Pro Ala Asp Ile Arg Arg Ala Thr Glu Ala Asp
   1               5                   10                  15
   Met Pro Ala Val Cys Thr Ile Val Asn His Tyr Ile Glu Thr Ser Thr
               20                  25                  30
   Val Asn Phe Arg Thr Glu Pro Gln Glu Pro Gln Glu Trp Thr Asp Asp
           35                  40                  45
   Leu Val Arg Leu Arg Glu Arg Tyr Pro Trp Leu Val Ala Glu Val Asp
       50                  55                  60
   Gly Glu Val Ala Gly Ile Ala Tyr Ala Gly Pro Trp Lys Ala Arg Asn
   65                  70                  75                  80
   Ala Tyr Asp Trp Thr Ala Glu Ser Thr Val Tyr Val Ser Pro Arg His
                   85                  90                  95
   Gln Arg Thr Gly Leu Gly Ser Thr Leu Tyr Thr His Leu Leu Lys Ser
               100                 105                 110
   Leu Glu Ala Gln Gly Phe Lys Ser Val Val Ala Val Ile Gly Leu Pro
           115                 120                 125
   Asn Asp Pro Ser Val Arg Met His Glu Ala Leu Gly Tyr Ala Pro Arg
       130                 135                 140
   Gly Met Leu Arg Ala Ala Gly Phe Lys His Gly Asn Trp His Asp Val
   145                 150                 155                 160
   Gly Phe Trp Gln Leu Asp Phe Ser Leu Pro Val Pro Pro Arg Pro Val
                   165                 170                 175
   Leu Pro Val Thr Glu Ile
               180
<210> 125
   <211> 366
   <212> DNA
   <213> Human
<220>
   <221> CDS
   <222> (1)..(363)
<400> 125
   atg gga aaa ggt gtg aaa tcc ccg ggg gag aag tca cgc tat gag acc        48
   Met Gly Lys Gly Val Lys Ser Pro Gly Glu Lys Ser Arg Tyr Glu Thr
   1               5                   10                  15
   tca ctg aat ctg acc acc aag cgc ttc ctg gag ctg ctg agc cac tcg        96
   Ser Leu Asn Leu Thr Thr Lys Arg Phe Leu Glu Leu Leu Ser His Ser
               20                  25                  30
   gct gac ggt gtc gtc gac ctg aac tgg gct gcc gag gtg ctg aag gtg       144
   Ala Asp Gly Val Val Asp Leu Asn Trp Ala Ala Glu Val Leu Lys Val
           35                  40                  45
   cag aag cgg cgc atc tat gac atc acc aac gtc ctt gag ggc atc cag       192
   Gln Lys Arg Arg Ile Tyr Asp Ile Thr Asn Val Leu Glu Gly Ile Gln
       50                  55                  60
   ctc att gcc aag aag tcc aag aac cac atc cag tgg ctg ggc agc cac       240
   Leu Ile Ala Lys Lys Ser Lys Asn His Ile Gln Trp Leu Gly Ser His
   65                  70                  75                  80
   acc aca gtg ggc gtc ggc gga cgg ctt gag ggg ttg acc cag gac ctc       288
   Thr Thr Val Gly Val Gly Gly Arg Leu Glu Gly Leu Thr Gln Asp Leu
                   85                  90                  95
   cga cag ctg cag gag agc gag cag cag ctg gac cac ctg atg aat atc       336
   Arg Gln Leu Gln Glu Ser Glu Gln Gln Leu Asp His Leu Met Asn Ile
               100                 105                 110
   tgt act acg cag ctg cgc ctg ctc tcc tga                               366
   Cys Thr Thr Gln Leu Arg Leu Leu Ser
           115                 120
<210> 126
   <211> 121
   <212> PRT
   <213> Human
<400> 126
   Met Gly Lys Gly Val Lys Ser Pro Gly Glu Lys Ser Arg Tyr Glu Thr
   1               5                   10                  15
   Ser Leu Asn Leu Thr Thr Lys Arg Phe Leu Glu Leu Leu Ser His Ser
               20                  25                  30
   Ala Asp Gly Val Val Asp Leu Asn Trp Ala Ala Glu Val Leu Lys Val
           35                  40                  45
   Gln Lys Arg Arg Ile Tyr Asp Ile Thr Asn Val Leu Glu Gly Ile Gln
       50                  55                  60
   Leu Ile Ala Lys Lys Ser Lys Asn His Ile Gln Trp Leu Gly Ser His
   65                  70                  75                  80
   Thr Thr Val Gly Val Gly Gly Arg Leu Glu Gly Leu Thr Gln Asp Leu
                   85                  90                  95
   Arg Gln Leu Gln Glu Ser Glu Gln Gln Leu Asp His Leu Met Asn Ile
               100                 105                 110
   Cys Thr Thr Gln Leu Arg Leu Leu Ser
           115                 120

## Description

### FIELD OF THE INVENTION

The present invention relates generally to gene expression. More particularly, the present invention relates to a method for modulating the quality of a selected phenotype that is displayed by an organism or part thereof and that results from the expression of a polynucleotide that encodes the polypeptide by replacing at least one codon of that polynucleotide with a synonymous codon that has a higher or lower preference of usage by the organism or part thereof to produce the selected phenotype than the codon it replaces. Even more particularly, the invention relates to the use of a protein-encoding polynucleotide whose codon composition has been modified for modulating the quality of a selected phenotype displayed by an organism or part thereof.

### BACKGROUND OF THE INVENTION

The expression of foreign heterologous genes in transformed cells is now commonplace. A large number of mammalian genes, including, for example, murine and human genes, have been successfully expressed in various host cells, including bacterial, yeast, insect, plant and mammalian host cells. Nevertheless, despite the burgeoning knowledge of expression systems and recombinant DNA technology, significant obstacles remain when one attempts to express a foreign or synthetic gene in a selected host cell. For example, translation of a synthetic gene, even when coupled with a strong promoter, often proceeds much more slowly than would be expected. The same is frequently true of exogenous genes that are foreign to the host cell. This lower than expected translation efficiency is often due to the protein coding regions of the gene having a codon usage pattern that does not resemble those of highly expressed genes in the host cell. It is known in this regard that codon utilisation is highly biased and varies considerably in different organisms and that biases in codon usage can alter peptide elongation rates. It is also known that codon usage patterns are related to the relative abundance of tRNA isoacceptors, and that genes encoding proteins of high *versus* low abundance show differences in their codon preferences.

The implications of codon preference phenomena on gene expression are manifest in that these phenomena can affect the translational efficiency of messenger RNA (mRNA). It is widely known in this regard that translation of "rare codons", for which the corresponding iso-tRNA is in low abundance relative to other iso-tRNAs, may cause a ribosome to pause during translation which can lead to a failure to complete a nascent polypeptide chain and an uncoupling of transcription and translation. Thus, the expression of an exogenous gene may be impeded severely if a particular host cell of an organism or the organism itself has a low abundance of iso-tRNAs corresponding to one or more codons of the exogenous gene. Accordingly, a major aim of investigators in this field is to first ascertain the codon preference for particular cells in which an exogenous gene is to be expressed, and to subsequently alter the codon composition of that gene for optimised expression in those cells.
WO98/12207 describes genes and methods for high level expression of proteins. WO01/66739 describes a cDNA encoding a TRAG gene (TGF-β resistance associated gene) and its protein product. US-A-5 689 052 describes synthetic DNA sequences having enhanced expression in monocotyledonous plants and method for preparation thereof.

Codon-optimisation techniques are known for improving the translational kinetics of translationally inefficient protein coding regions. Traditionally, these techniques have been based on the replacement of codons that are *rarely* or *infrequently* used in the host cell with those that are *host-preferred.* Codon frequencies can be derived from literature sources for the highly expressed genes of many organisms (see, for example, Nakamura et al., 1996, Nucleic Acids Res 24: 214-215). These frequencies are generally expressed on an 'organism-wide average basis' as the percentage of occasions that a synonymous codon is used to encode a corresponding amino acid across a collection of protein-encoding genes of that organism, which are preferably highly expressed.

Typically, codons are classified as: (a) "common" codons (or "preferred" codons) if their frequency of usage is above about 4/3 x the frequency of usage that would be expected in the absence of any bias in codon usage; (b) "rare" codons (or "non-preferred" codons) if their frequency of usage is below about 2/3 x the frequency of usage that would be expected in the absence of any bias in codon usage; and (c) "intermediate" codons (or "less preferred" codons) if their frequency of usage is in-between the frequency of usage of "common" codons and of "rare" codons. Since an amino acid can be encoded by 2, 3, 4 or 6 codons, the frequency of usage of any selected codon, which would be expected in the absence of any bias in codon usage, will be dependent upon the number of synonymous codons which code for the same amino acid as the selected codon. Accordingly, for a particular amino acid, the frequency thresholds for classifying codons in the "common", "intermediate" and "rare" categories will be dependent upon the number of synonymous codons for that amino acid. Consequently, for amino acids having 6 choices of synonymous codon, the frequency of codon usage that would be expected in the absence of any bias in codon usage is 16% and thus the "common", "intermediate" and "rare" codons are defined as those codons that have a frequency of usage above 20%, between 10 and 20% and below 10%, respectively. For amino acids having 4 choices of synonymous codon, the frequency of codon usage that would be expected in the absence of codon usage bias is 25% and thus the "common", "intermediate" and "rare" codons are defined as those codons that have a frequency of usage above 33%, between 16 and 33% and below 16%, respectively. For isoleucine, which is the only amino acid having 3 choices of synonymous codon, the frequency of codon usage that would be expected in the absence of any bias in codon usage is 33% and thus the "common", "intermediate" and "rare" codons for isoleucine are defined as those codons that have a frequency of usage above 45%, between 20 and 45% and below 20%, respectively. For amino acids having 2 choices of synonymous codon, the frequency of codon usage that would be expected in the absence of codon usage bias is 50% and thus the "common", "intermediate" and "rare" codons are defined as those codons that have a frequency of usage above 60%, between 30 and 60% and below 30%, respectively. Thus, the categorisation of codons into the "common", "intermediate" and "rare" classes (or "preferred", "less preferred" or "non preferred", respectively) has been based conventionally on a compilation of codon usage for an organism in general (*e.g*., 'human-wide') or for a class of organisms in general (e.g., 'mammal-wide'). For example, reference may be made to Seed (see U.S. Patent Serial Nos 5,786,464 and 5,795,737) who discloses preferred, less preferred and non-preferred codons for mammalian cells in general. However, the present inventor revealed in WO 99/02694 and in WO 00/42190 that there are substantial differences in the relative abundance of particular iso-tRNAs in different cells or tissues of a single multicellular organism (e.g., a mammal or a plant) and that this plays a pivotal role in protein translation from a coding sequence with a given codon usage or composition.

Thus, in contrast to the art-recognised presumption that different cells of a multicellular organism have the same bias in codon usage, it was revealed for the first time that one cell type of a multicellular organism uses codons in a manner distinct from another cell type of the same organism. In other words, it was revealed that different cells of an organism can exhibit different translational efficiencies for the same codon and that it was not possible to predict which codons would be preferred, less preferred or non preferred in a selected cell type. Accordingly, it was proposed that differences in codon translational efficiency between cell types could be exploited, together with codon composition of a gene, to regulate the production of a protein in, or to direct that production to, a chosen cell type.

Therefore, in order to optimise the expression of a protein-encoding polynucleotide in a particular cell type, it is necessary to first determine the translational efficiency for each codon in that cell type, rather than to rely on codon frequencies calculated on an organism-wide average basis, and then to codon modify the polynucleotide based on that determination.

All of the above methods relate to the codon optimisation of protein-encoding polynucleotides for modulating the expression of those polynucleotides in a chosen cell type or for differentially expressing those polynucleotides between selected cell types.

### BRIEF SUMMARY OF THE INVENTION

The present invention relates to a novel strategy for changing the quality of a selected phenotype that is displayed by an organism of interest and that results from the expression of a polynucleotide that encodes a polypeptide. In contrast to methods heretofore described, this strategy does not rely on codon usage data or on codon translational efficiency data that may be applicable to the organism of interest. Instead, it relies on ranking individual synonymous codons that code for an amino acid in the polypeptide according to their preference of usage by the organism of interest for producing the selected phenotype. In other words, the subject method is based on determining the "phenotypic preferences" of individual synonymous codons. Advantageously, phenotypic preferences can be determined by introducing into the organism of interest or into a related organism a synthetic construct that comprises a regulatory polynucleotide operably linked to a tandem repeat of a codon fused in frame with a reporter polynucleotide that encodes a protein, which produces, or which is predicted to produce, the selected phenotype or a phenotype of the same class as the selected phenotype. The quality of the phenotype produced by the expression of the synthetic construct in the organism of interest or in the related organism is then determined using a suitable assay. The selected phenotype may be a therapeutic or prophylactic phenotype including immunity, tolerance, pathogen resistance etc, or other beneficial trait including enhancement or prevention of a repair process, pest resistance, frost resistance, herbicide tolerance etc. In accordance with the present invention, a set of synonymous codons will often display a range of phenotypic preferences, which can be used as a basis for rationally selecting a codon in the original polynucleotide for replacement with a synonymous codon that has a different phenotypic preference.

Thus in one aspect of the present invention, there is provided a method for constructing a synthetic polynucleotide from which a polypeptide is producible to confer a selected phenotype upon a multicellular organism of interest or part thereof in a different quality than that conferred by a parent polynucleotide that encodes the same polypeptide, the method comprising: (a) selecting a first codon of the parent polynucleotide for replacement with a synonymous codon, wherein the synonymous codon is selected on the basis that it exhibits a different phenotypic preference than the first codon in a comparison of phenotypic preferences in non-human test organisms or parts thereof, wherein the parts of test organisms are selected from tissues or organs of the test organisms, wherein the test organisms are selected from the group consisting of organisms of the same species as the organism of interest and organisms that are related to the organism of interest: and (b) replacing the first codon with the synonymous codon to construct the synthetic polynucleotide, wherein the selected phenotype is other than a phenotype conferred upon a cell by the parent polynucleotide containing the first codon that is expressed in the cell and that encodes the polypeptide.
In a further aspect of the present invention, there is provided a method for constructing a synthetic polynucleotide from which a polypeptide is producible to confer a selected phenotype upon a multicellular organism of interest or part thereof in a different quality than that conferred by a parent polynucleotide that encodes the same polypeptide, the method comprising: (a) selecting a first codon of the parent polynucleotide for replacement with a synonymous codon, wherein the synonymous codon is selected on the basis that it exhibits a different phenotypic preference than the first codon in a comparison of phenotypic preferences in *ex vivo* parts of test organisms, wherein said parts of test organisms are selected from tissues or organs of the test organisms, wherein said parts of test organisms do not include a human embryonic stem cell or a human germ line cell, wherein the parts of test organisms are selected from the group consisting of organisms of the same species as the organism of interest and organisms that are related to the organism of interest: and (b) replacing the first codon with the synonymous codon to construct the synthetic polynucleotide, wherein the selected phenotype is other than a phenotype conferred upon a cell by the parent polynucleotide containing the first codon that is expressed in the cell and that encodes the polypeptide.

The phenotypic preferences of codons in the test organisms or parts are suitably compared by: (i) separately introducing into the test organisms or parts individual synthetic constructs, each of which comprises a regulatory polynucleotide operably linked to a tandem repeat of a codon fused in frame with a reporter polynucleotide that encodes a reporter protein, which produces, or which is predicted to produce, a corresponding phenotype selected from the group consisting of the selected phenotype and a phenotype of the same class as the selected phenotype; and (ii) comparing the quality of the phenotypes displayed by the test organisms or parts to determine the relative phenotypic preferences of the codons.

The synthetic constructs are typically introduced into the test organisms or parts using the same or similar mode of introduction. This is desirable when the corresponding phenotype or its quality is dependent on a particular mode or site of introduction of the synthetic constructs.

In some embodiments, the tandem repeat of each of the synthetic constructs comprises at least three copies of the corresponding codon.

In some embodiments, the synonymous codon is selected such that it has a higher phenotypic preference than the first codon. In accordance with the present invention, a higher phenotypic preference will correlate with a higher quality of the selected phenotype. Accordingly, a synonymous codon for these embodiments is preferably selectable when the quality of the phenotype conferred by the synthetic construct comprising a tandem repeat of the synonymous codon is suitably at least about 5% higher than the quality of the phenotype conferred by the synthetic construct comprising a tandem repeat of the first codon.

In other embodiments, the synonymous codon is selected such that it has a lower phenotypic preference than the first codon. According to the subject invention, a lower phenotypic preference will correlate with a lower quality of the selected phenotype. Thus, a synonymous codon for these embodiments is preferably selectable when the quality of the phenotype conferred by the synthetic construct comprising a tandem repeat of the synonymous codon is suitably at least about 5% lower than the quality of the phenotype conferred by the synthetic construct comprising a tandem repeat of the first codon.

The present invention is applicable to multicellular organisms including plants and animals.

In another aspect, the invention provides a method for determining the phenotypic preference of a first codon in a multicellular organism of interest pr part thereof, the method comprising: (a) introducing a synthetic construct into a non-human test organism or part thereof, wherein said part of a test organism is selected from tissues or organs of the test organism, wherein the test organism is selected from the group consisting of an organism of the same species as the organism of interest and an organism that is related to the organism of interest, the synthetic construct comprising a regulatory polynucleotide operably linked to a tandem repeat of the first codon fused in frame with a reporter polynucleotide that encodes a reporter protein, which produces, or which is predicted to produce, a selected phenotype or a phenotype of the same class as the selected phenotype; and (b) determining the quality of the corresponding phenotype displayed by the non-human test organism or part, wherein the selected phenotype or the phenotype of the same class as the selected phenotype is other than a phenotype conferred upon a cell by the parent polynucleotide containing the first codon that is expressed in the cell and that encodes the polypeptide.
In a further aspect of the preset invention, there is provided a method for determining the phenotypic preference of a first codon in a multicellular organism of interest or part thereof, the method comprising: (a) introducing a synthetic construct into an *ex vivo* part of a test organism, wherein said part of a test organism is selected from tissues or organs of the test organism, wherein said part of a test organism does not include a human embryonic stem cell or a human germ line cell, wherein the part of a test organism is selected from the group consisting of an organism of the same species as the organism of interest and an organism that is related to the organism of interest, the synthetic construct comprising a regulatory polynucleotide operably linked to a tandem repeat of the first codon fused in frame with a reporter polynucleotide that encodes a reporter protein, which produces, or which is predicted to produce, a selected phenotype or a phenotype of the same class as the selected phenotype; and (b) determining the quality of the corresponding phenotype displayed by the *ex vivo* part of a test organism, wherein the selected phenotype or the phenotype of the same class as the selected phenotype is other than a phenotype conferred upon a cell by the parent polynucleotide containing the first codon that is expressed in the cell and that encodes the polypeptide.
In some embodiments, the method further comprises comparing (i) the quality of the corresponding phenotype displayed by an *ex vivo* part of a test organism to which a synthetic construct comprising a tandem repeat of the first codon was provided, wherein said part of a test organism is selected from tissues or organs of the test organism, wherein said part of a test organism does not include a human embryonic stem cell or a human germ line cell; and (ii) the quality of the corresponding phenotype displayed by an *ex vivo* part of a test organism to which a synthetic construct comprising a tandem repeat of a second codon was provided, wherein said part of a test organism is selected from tissues or organs of the test organism, wherein said part of a test organism does not include a human embryonic stem cell or a human germ line cell, wherein the second codon encodes the same amino acid as the first codon, to thereby determine the phenotypic preference of the first codon relative to the phenotypic preference of the second codon in the test organism or part.

In some embodiments, the methods further comprise: comparing (i) the quality of the corresponding phenotype displayed by a test organism or part to which a synthetic construct comprising a tandem repeat of the first codon was provided; and (ii) the quality of the corresponding phenotype displayed by a test organism or part to which a synthetic construct comprising a tandem repeat of a second codon was provided, wherein the second codon encodes the same amino acid as the first codon, to thereby determine the phenotypic preference of the first codon relative to the phenotypic preference of the second codon in the test organism or part.

In some embodiments, the methods further comprise: (1) introducing the synthetic construct into a progenitor of a test organism or part; and (2) producing the test organism or part from the progenitor, wherein the test organism or part contains the synthetic construct.

In other embodiments, the methods further comprise: (1) introducing the synthetic construct into a progenitor of a test organism or part; and (2) growing the test organism or part from the progenitor; wherein the test organism or part comprises a cell containing the synthetic construct.

In still other embodiments, the methods further comprise: introducing the synthetic construct into a selected cell of the test organism or part.

A synthetic polynucleotide may be constructed according to any one of the above methods.

We describe an organism of interest or part thereof containing a synthetic polynucleotide constructed according to any one of the above methods.

An organism of interest or part thereof may contain a synthetic construct that comprises a regulatory polynucleotide operably linked to a tandem repeat of a first codon fused in frame with a reporter polynucleotide that encodes a reporter protein, which produces, or which is predicted to produce, a selected phenotype or a phenotype of the same class as the selected phenotype.

We describe methods of modulating the quality of a selected phenotype that is displayed by an organism of interest or part thereof and that results from the expression of a parent polynucleotide that encodes the polypeptide. These methods generally comprise: introducing into the organism or part a synthetic polynucleotide that is distinguished from the parent polynucleotide by the replacement of a first codon in the parent polynucleotide with a synonymous codon that exhibits a different phenotypic preference than the first codon in the organism or part.

We describe methods of enhancing the quality of a selected phenotype that is displayed by an organism of interest or part thereof and that results from the expression of a parent polynucleotide that encodes the polypeptide. These methods generally comprise: introducing into the organism or part a synthetic polynucleotide that is distinguished from the parent polynucleotide by the replacement of a first codon in the parent polynucleotide with a synonymous codon that exhibits a higher phenotypic preference than the first codon in the organism or part.

We describe methods of reducing the quality of a selected phenotype that is displayed by an organism of interest or part thereof and that results from the expression of a parent polynucleotide that encodes the polypeptide. These methods generally comprise: introducing into the organism or part a synthetic polynucleotide that is distinguished from the parent polynucleotide by the replacement of a first codon in the parent polynucleotide with a synonymous codon that exhibits a lower phenotypic preference than the first codon in the organism or part.

### DETAILED DESCRIPTION OF THE INVENTION

### 1. Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles *"a"* and *"an"* are used herein to refer to one or to more than one (i.e., to at least one) of the grammatical object of the article. By way of example, "an element" means one element or more than one element.

The term "*about*" is used herein to refer to parameters (e.g., amounts, concentrations, phenotype-conferring efficiencies, time etc) that vary by as much as 30%, 20%, 15%, 10% , 5% or even 4%, 3%, 2%, 1% to a specified parameter.

As used herein, the term "*cis-acting sequence"* or "*cis-regulatory region"* or similar term shall be taken to mean any sequence of nucleotides which is derived from an expressible genetic sequence wherein the expression of the genetic sequence is regulated, at least in part, by said sequence of nucleotides. Those skilled in the art will be aware that a *cis*-regulatory region may be capable of activating, silencing, enhancing, repressing or otherwise altering the level of expression and/or cell-type-specificity and/or developmental specificity of any structural gene sequence.

Throughout this specification, unless the context requires otherwise, the words "*comprise*", "*comprises*" and "*comprising*" will be understood to imply the inclusion of a stated step or element or group of steps or elements but not the exclusion of any other step or element or group of steps or elements.

As used herein a "*conferred phenotype*" refers to a temporary or permanent change in the state of an organism of interest or class of organisms of interest, or of a part or tissue or cell or cell type or class of cell of an organism of interest, which occurs after the introduction of a polynucleotide to that organism, or to that class of organisms, or to the part or tissue or cell or cell type or class of cell, or to a precursor of that organism or part or tissue or cell or cell type or class of cell, and which would not have occurred in the absence of that introduction. Typically, such a temporary or permanent change occurs as a result of the transcription and/or translation of genetic information contained within that polynucleotide in the cell, or in at least one cell or cell type or class of cell within the organism of interest or within the class of class of organisms of interest, and can be used to distinguish the organism of interest, or class of organisms of interest, or part or tissue or cell or cell type or class of cell thereof, or genetic progeny of these, to which the polynucleotide has been provided from a similar organism of interest, or class of organisms of interest, or part or tissue or cell or cell type or class of cell thereof, or genetic progeny of these, to which the polynucleotide has not been provided.

By "*expression vector*" is meant any autonomous genetic element capable of directing the synthesis of a protein encoded by the vector. Such expression vectors are known by practitioners in the art.

The term "*gene*" as used herein refers to any and all discrete coding regions of a host genome, or regions that code for a functional RNA only (e.g., tRNA, rRNA, regulatory RNAs such as ribozymes, post translational gene silencing-associated RNAs etc) as well as associated non-coding regions and optionally regulatory regions. In certain embodiments, the term "gene" gene includes within its scope the open reading frame encoding specific polypeptides, introns, and adjacent 5' and 3' non-coding nucleotide sequences involved in the regulation of expression. In this regard, the gene may further comprise control signals such as promoters, enhancers, termination and/or polyadenylation signals that are naturally associated with a given gene, or heterologous control signals. The gene sequences may be cDNA or genomic DNA or a fragment thereof. The gene may be introduced into an appropriate vector for extrachromosomal maintenance or for integration into the host.

By "*modulating*", "*modulate*" and the like is meant increasing or decreasing, either directly or indirectly, the quality of a selected phenotype.

By *"natural gene*" is meant a gene that naturally encodes the protein. However, it is possible that the parent polynucleotide encodes a protein that is not naturally-occurring but has been engineered using recombinant techniques.

The term "*5*' *non-coding region"* is used herein in its broadest context to include all nucleotide sequences which are derived from the upstream region of an expressible gene, other than those sequences which encode amino acid residues which comprise the polypeptide product of said gene, wherein 5' non-coding region confers or activates or otherwise facilitates, at least in part, expression of the gene.

The term *"oligonucleotide"* as used herein refers to a polymer composed of a multiplicity of nucleotide units (deoxyribonucleotides or ribonucleotides, or related structural variants or synthetic analogues thereof) linked *via* phosphodiester bonds (or related structural variants or synthetic analogues thereof). Thus, while the term "oligonucleotide" typically refers to a nucleotide polymer in which the nucleotides and linkages between them are naturally occurring, it will be understood that the term also includes within its scope various analogues including, but not restricted to, peptide nucleic acids (PNAs), phosphoramidates, phosphorothioates, methyl phosphonates, 2-O-methyl ribonucleic acids, and the like. The exact size of the molecule may vary depending on the particular application. An oligonucleotide is typically rather short in length, generally from about 10 to 30 nucleotides, but the term can refer to molecules of any length, although the term "polynucleotide" or "nucleic acid" is typically used for large oligonucleotides.

The term "*operably connected*" or "*operably linked*" as used herein means placing a structural gene under the regulatory control of a promoter, which then controls the transcription and optionally translation of the gene. In the construction of heterologous promoter/structural gene combinations, it is generally preferred to position the genetic sequence or promoter at a distance from the gene transcription start site that is approximately the same as the distance between that genetic sequence or promoter and the gene it controls in its natural setting; i.e. the gene from which the genetic sequence or promoter is derived. As is known in the art, some variation in this distance can be accommodated without loss of function. Similarly, the preferred positioning of a regulatory sequence element with respect to a heterologous gene to be placed under its control is defined by the positioning of the element in its natural setting; i.e. the genes from which it is derived.

The terms "*precursor*" and "*progenitor*" as used herein refer to a cell or part of organism that can gives rise to an organism of interest in which phenotypic expression is desired or in which phenotypic preference of a codon is to be determined.

The term "*phenotype*" means any one or more detectable physical or functional characteristics, properties, attributes or traits of an organism, tissue, or cell, or class of organisms, tissues or cells, which generally result from the interaction between the genetic makeup (i.e., genotype) of the organism, tissue, or cell, or the class of organisms, tissues or cells and the environment. In certain embodiments, the term "phenotype" excludes resistance to a selective agent or screening an enzymic or light-emitting activity, conferred directly by a reporter protein.

By "*phenotypic preference*" is meant the preference with which an organism uses a codon to produce a selected phenotype. This preference can be evidenced, for example, by the quality of a selected phenotype that is producible by a polynucleotide that comprises the codon in an open reading frame which codes for a polypeptide that produces the selected phenotype. In certain embodiment, the preference of usage is independent of the route by which the polynucleotide is introduced into the organism. However, in other embodiments, the preference of usage is dependent on the route of introduction of the polynucleotide into the organism.

The term "*polynucleotide*" or *"nucleic acid*" as used herein designates mRNA, RNA, cRNA, cDNA or DNA. The term typically refers to oligonucleotides greater than 30 nucleotides in length.

"*Polypeptide*", "*peptide*" and "*protein*" are used interchangeably herein to refer to a polymer of amino acid residues and to variants and synthetic analogues of the same. Thus, these terms apply to amino acid polymers in which one or more amino acid residues is a synthetic non-naturally occurring amino acid, such as a chemical analogue of a corresponding naturally occurring amino acid, as well as to naturally-occurring amino acid polymers.

By "*producing*"*,* and like terms such as "*production*" and "*producible*", in the context or protein production, is meant production of a protein to a level sufficient to achieve a particular function or phenotype associated with the protein. By contrast, the terms *"not producible"* and *"not substantially producible*" as used interchangeably herein refer to (a) no production of a protein, (b) production of a protein to a level that is not sufficient to effect a particular function or phenotype associated with the protein, (c) production of a protein, which cannot be detected by a monoclonal antibody specific for the protein, or (d) production of a protein, which is less that 1% of the level produced in a wild-type cell that normally produces the protein.

Reference herein to a "*promoter*" is to be taken in its broadest context and includes the transcriptional regulatory sequences of a classical genomic gene, including the TATA box which is required for accurate transcription initiation, with or without a CCAAT box sequence and additional regulatory elements (i.e. upstream activating sequences, enhancers and silencers) which alter gene expression in response to developmental and/or environmental stimuli, or in a tissue-specific or cell-type-specific manner. A promoter is usually, but not necessarily, positioned upstream or 5', of a structural gene, the expression of which it regulates. Furthermore, the regulatory elements comprising a promoter are usually positioned within 2 kb of the start site of transcription of the gene. Preferred promoters according to the invention may contain additional copies of one or more specific regulatory elements to further enhance expression in a cell, and/or to alter the timing of expression of a structural gene to which it is operably connected.

The term "*quality*" is used herein in its broadest sense and includes a measure, strength, intensity, degree or grade of a phenotype, e.g., a superior or inferior immune response, increased or decreased disease resistance, higher or lower sucrose accumulation, better or worse salt tolerance etc.

By "*recombinant polypeptide*" is meant a polypeptide made using recombinant techniques, i.e., through the expression of a recombinant or synthetic polynucleotide.

The term "*synthetic polynucleotide*" as used herein refers to a polynucleotide formed *in vitro* by the manipulation of a polynucleotide into a form not normally found in nature. For example, the synthetic polynucleotide can be in the form of an expression vector. Generally, such expression vectors include transcriptional and translational regulatory polynucleotide operably linked to the polynucleotide.

The term "*synonymous codon"* as used herein refers to a codon having a different nucleotide sequence than another codon but encoding the same amino acid as that other codon.

By *"vector"* is meant a nucleic acid molecule, preferably a DNA molecule derived, for example, from a plasmid, bacteriophage, or plant virus, into which a nucleic acid sequence may be inserted or cloned. A vector preferably contains one or more unique restriction sites and may be capable of autonomous replication in a defined host cell including a target cell or tissue or a progenitor cell or tissue thereof, or be integrable with the genome of the defined host such that the cloned sequence is reproducible. Accordingly, the vector may be an autonomously replicating vector, i.e., a vector that exists as an extrachromosomal entity, the replication of which is independent of chromosomal replication, e.g., a linear or closed circular plasmid, an extrachromosomal element, a minichromosome, or an artificial chromosome. The vector may contain any means for assuring self-replication. Alternatively, the vector may be one which, when introduced into the host cell, is integrated into the genome and replicated together with the chromosome(s) into which it has been integrated. A vector system may comprise a single vector or plasmid, two or more vectors or plasmids, which together contain the total DNA to be introduced into the genome of the host cell, or a transposon. The choice of the vector will typically depend on the compatibility of the vector with the host cell into which the vector is to be introduced. The vector may also include a selection marker such as an antibiotic resistance gene that can be used for selection of suitable transformants. Examples of such resistance genes are well known to those of skill in the art.

### 2. Phenotypic preference of codons

The present invention provides a novel strategy for enhancing or reducing the quality of a selected phenotype that is displayed, or proposed to be displayed, by an organism of interest. The strategy involves codon modification of a polynucleotide that encodes a phenotype-associated polypeptide that either by itself or in association with other molecules, in the organism of interest imparts or confers the selected phenotype upon the organism. Unlike previous methods, however, the strategy does not rely on data that provide a ranking of synonymous codons according to their preference of usage in an organism or class of organisms. Nor does it rely on data that provide a ranking of synonymous codons according to their translational efficiencies in one or more cells of the organism or class of organisms. Instead, it relies on ranking individual synonymous codons that code for an amino acid in the phenotype-associated polypeptide according to their preference of usage by the organism or class of organisms, or by a part thereof, for producing the selected phenotype. As used herein, the preference of usage of an individual codon by an organism or class of organisms or by a part thereof is referred to as a "*phenotypic preference*". Such preference may be determined by qualitatively or quantitatively determining the influence of the individual codon on a achieving the selected phenotype and it does not require, therefore, the calculation of codon frequencies or translational efficiencies as heretofore described.

Advantageously, phenotypic preferences for synonymous codons can be determined by introducing into the organism of interest or into a related organism, or into a part of such organisms a synthetic construct that comprises a regulatory polynucleotide operably linked to a tandem repeat of a codon fused in frame with a reporter polynucleotide that encodes a reporter protein, which produces, or which is predicted to produce, the selected phenotype or a phenotype of the same class as the selected phenotype. In one embodiment, the reporter protein is a phenotype-associated polypeptide (e.g., a melanoma specific antigen such as BAGE or GAGE-1) that will be the subject of producing the selected phenotype (e.g., immunity to melanoma). In another embodiment, the phenotype-associated polypeptide (e.g., green fluorescent protein or a gastrointestinal associated antigen such as 17-1A) is incapable of producing the selected phenotype (e.g., immunity to melanoma) but produces the same class of phenotype (e.g., an immune response) as the selected phenotype. In illustrative examples, the phenotype-associated polypeptide is selected from antigens including antigens from pathogenic organisms or cancers (e.g., wherein the phenotype is immunity to disease) and self antigens or transplantation antigens (e.g., wherein the phenotype is antigen-specific anergy or tolerance), growth factors (e.g., wherein the phenotype is selected from size of the organism or part, wound healing, cell proliferation, cell differentiation, cell migration, immune cell function), hormones (e.g., wherein the phenotype is increased lactation, e.g., using oxytocin, or amelioration of a diabetic state, e.g., using insulin) and toxins (e.g., wherein the phenotype is tumour regression or cell death).

The design of the synthetic construct is preferably based on the synthetic construct design of Frazer *et al.* in International Publication WO 00/42215, which is predicated on the determination that different but synonymous stretches of identical codons fused respectively in frame with a reporter polynucleotide can give rise to different levels of reporter protein produced within a given cell type. The level of reporter protein produced in a selected cell type is sensitive to the intracellular abundance of the iso-tRNA species corresponding to the identical codons of the construct and, therefore, provide a direct correlation of a cell's preference for translating a given codon. However, in contrast to WO 00/42215, which is primarily concerned with determining translational efficiencies of synonymous codons in selected cell types, the present invention is concerned with using synthetic constructs to determine the influence of their synonymous stretches of identical codons on the phenotype or class of phenotype displayed by the organism or part in response to the phenotype-associated protein produced by those synthetic constructs. This means, for example, that if the quality of the phenotype displayed by a selected organism or part thereof to which a synthetic construct having a tandem series of identical first codons is provided is lower than the quality of the phenotype displayed by the selected organism or part to which a synthetic construct having a tandem series of identical second codons is provided (i.e., wherein the first codons are different than, but synonymous with, the second codons), then it can be deduced that the selected organism or part has a higher preference for the second codon than the first codon with respect to the quality of the phenotype produced. Put another way, the second codon has a higher phenotypic preference than the first codon in the selected organism or part.

Suitably, the tandem repeat comprises at least three identical codons, preferably between four and seven identical codons and more preferably five or six identical codons.

The tandem repeat can be fused at a location adjacent to, or within, the reporter polynucleotide. This location is preferably selected such that the tandem repeat interferes with translation of at least a portion of the phenotype-associated protein. Preferably, the tandem repeat is located immediately upstream (translationally) from the reporter polynucleotide.

It is of course possible that a tandem repeat of identical amino acid residues (e.g., an oligo-proline repeat) can render the phenotype-associated protein unstable. Typically, protein instability is detected when the phenotype conferred by expression of the reporter polynucleotide is not detectable with any choice of isoaccepting codon specific for the amino acid corresponding to the tandem repeat. However, such protein instability can be alleviated by use of at least one spacer codon within the tandem repeat of identical codons, wherein the spacer codon encodes a neutral amino acid.

The spacer codon(s) can be placed adjacent to, or interposed between, some or all of the identical codons corresponding to the tandem repeat. For example, a suitable interposition for a penta-repeat of identical codons can be selected from the group consisting of: (a) I-S-I-S-I-S-I-S-IS; (b) S-I-S-I-S-I-S-I-S-I; (c) I-S-I-S-I-I-S-I; (d) I-S-I-I-S-I-S-I; (e) I-S-I-S-I-I-I; (f) I-I-S-I-S-I-I; (g) I-I-I-S-I-S-I; (h) I-S-I-I-S-I-I; (i) I-I-S-I-I-S-I; (j) I-S-I-I-I-S-I; (k) I-S-I-I-I-I; (l) I-I-S-I-I-I; (m) I-I-I-S-I-I; and (n) I-I-I-I-S-I, wherein I corresponds to an identical codon of a tandem repeat and S corresponds to a spacer codon.

Preferably, a spacer codon is efficiently translated in a cell type of the organism or the organism itself relative to other synonymous codons. This is important so that translation of the spacer codon is not rate limiting. The neutral amino acid includes, but is not restricted to, alanine and glycine.

The reporter polynucleotide can encode any suitable protein that confers upon the organism or part, either by itself or in association with other molecules, a phenotype or class of phenotype. In specific embodiments, the selected phenotype or class of phenotype corresponds to a beneficial or improved or superior state or condition of the organism or part thereof relative to a reference state or condition. In illustrative examples, the reference state or condition corresponds to a pathophysiological state. Phenotypes contemplated by the present invention include any desirable beneficial trait including, but not restricted to: immunity (e.g., immunity to pathogenic infection or cancer); antigen tolerance (e.g., antigen-specific T lymphocyte anergy, tolerance to allergens, transplantation antigens and self antigens); angiogenesis (e.g., blood vessel formation in the heart and vasculature and in tumour growths); anti-angiogenesis (e.g., treatment of ischaemic heart disease and tumours); amelioration of clinical symptoms (e.g., fever; inflammation; encephalitis; weight loss; anaemia; sensory symptoms such as paraesthesia or hypaesthesia; ataxia; neuralgia; paralysis; vertigo; urinary or bowel movement abnormalities; and cognitive dysfunction such memory loss, impaired attention, problem-solving difficulties, slowed information processing, and difficulty in shifting between cognitive tasks); reduced or increased cell death (e.g., apoptosis); reduced or increased cell differentiation; reduced or increased cell proliferation; tumour or cancer regression; growth and repair of tissue or organ; decreased fibrosis; inhibition or reversal of cell senescence; increased or reduced cell migration; differential expression of protein between different cells or tissues of an organism or part thereof; trauma recovery; recovery from burns; antibiotic resistance or sensitivity (e.g., resistance or sensitivity to aminoglycosidic antibiotics such as geneticin and paromomycin); herbicide tolerance or sensitivity (e.g. tolerance or sensitivity to glyphosate or glufosinate); starch biosynthesis or modification (e.g. using a starch branching enzyme, starch synthases, ADP-glucose pyrophosphorylase); fatty acid biosynthesis (e.g. using a desaturase or hydroxylase); disease resistance or tolerance (e.g., resistance to animal diseases such as cardiovascular disease, autoimmunity, Alzheimer's disease, Parkinson's disease, diabetes, AIDS etc or resistance to plant diseases such as rust, dwarfism, rot, smut, mould, scab and mildew); pest resistance or tolerance including insect resistance or tolerance (e.g., resistance to borers and worms); viral resistance or tolerance (e.g. resistance to animal viruses such as herpesviruses, hepadnaviruses, adenoviruses, flaviviruses, lentiviruses, poxviruses etc or resistance to plant viruses such as badnaviruses, caulimoviruses, potyviruses, luteoviruses, rhabdoviruses etc); fungal resistance or tolerance (e.g., resistance to arbuscular mycorrhizal fungi, endophytic fungi etc); a metabolic trait including sucrose metabolism (e.g., sucrose isomerisation); frost resistance or tolerance; stress tolerance (e.g., salt tolerance, drought tolerance); and improved food content or increased yields. Persons of skill in the art will recognise that the above exemplary classes of phenotype may be subdivided into phenotypic subclasses and that such subclasses would also fall within the scope of phenotypic classes contemplated by the present invention. For example, subclasses of immunity include innate immunity (which can be further subdivided *inter alia* into complement system, monocytes, macrophages, neutrophils and natural killer cells), cellular immunity (which can be further subdivided *inter alia* into cytolytic T lymphocytes, dendritic cells and T helper lymphocytes) and humoral immunity (which can be further subdivided *inter alia* into antibody subclasses IgA, IgD, IgE, IgG and IgM).

The instant method is applicable to multicellular organisms, including eukaryotic hosts such as, but not limited to yeast, plants and animals including vertebrate animals such as mammals, reptiles, fish, birds etc as well as invertebrate animals such as metazoa, sponges, worms, molluscs, nematodes, crustaceans, echinoderms etc. In certain embodiments of the present invention, the organism of interest is selected from plants and mammals.

Illustrative examples of eukaryotic organisms include, but are not limited to, fungi such as yeast and filamentous fungi, including species of *Aspergillus, Trichoderma,* and *Neurospora;* animal hosts including vertebrate animals illustrative examples of which include fish (e.g., salmon, trout, tulapia, tuna, carp, flounder, halobut, swordfish, cod and zebrafish), birds (e.g., chickens, ducks, quail, pheasants and turkeys, and other jungle foul or game birds) and mammals (e.g., dogs, cats, horses, cows, buffalo, deer, sheep, rabbits, rodents such as mice, rats, hamsters and guinea pigs, goats, pigs, primates, marine mammals including dolphins and whales, and non-human stem cells, including pluripotent and non-pluripotent and non-human embryonic stem cells, and non-human zygotes), as well as invertebrate animals illustrative examples of which include nematodes (representative generae of which include those that infect animals such as but not limited to *Ancylostoma, Ascaridia, Ascaris, Bunostomum, Caenorhabditis, Capillaria, Chabertia, Cooperia, Dictyocaulus, Haernonchus, Heterakis, Nematodirus, Oesophagostomum, Ostertagia, Oxyuris. Parascaris, Strongylus, Toxascaris, Trichuris, Trichostrongylus, Tflichonema, Toxocara, Uncinaria,* and those that infect plants such as but not limited to *Bursaphalenchus, Criconerriella, Diiylenchus, Ditylenchus, Globodera, Helicotylenchus, Heterodera, Longidorus, Melodoigyne, Nacobbus, Paratylenchus, Pratylenchus, Radopholus, Rotelynchus*, *Tylenchus,* and *Xiphinerna*) and other worms, drosophila, and other insects (such as from the families Apidae, Curculionidae, Scarabaeidae, Tephritidae, Tortricidae, amongst others, representative orders of which include Coleaptera, Diptera, Lepidoptera, and Homoptera.

In certain embodiments, the organism of interest is a plant which is suitably selected from monocotyledons, dicotyledons and gymnosperms. The plant may be an ornamental plant or crop plant. Illustrative examples of ornamental plants include, but are not limited to, *Malus* spp, *Crataegus* spp, *Rosa* spp., *Betula* spp, *Sorbus* spp, *Olea* spp, *Nerium* spp, *Salix* spp, *Populus* spp. Illustrative examples of crop plants include plant species which are cultivated in order to produce a harvestable product such as, but not limited to, *Abelmoschus esculentus* (okra). *Acacia* spp., *Agave fourcroydes* (henequen), *Agave sisalana* (sisal), *Albizia* spp., *Allium fistulosum* (bunching onion), *Allium sativum* (garlic), *Allium* spp. (onions), *Alpinia galanga* (greater galanga), *Amaranthus caudatus, Amarnthus* spp., *Anacardium* spp. (cashew), *Ananas comosus* (pineapple), *Anethum graveolens* (dill), *Annona cherimola* (cherimoya), *Apios americana* (American potatobean), *Arachis hypogaea* (peanut), *Arctium* spp. (burdock), *Artemisia* spp. (wormwood), *Aspalathus linearis* (redbush tea), *Athertonia diversifolia, Atriplex nummularia* (old man saltbush), *Averrhoa carambola* (starfruit), *Azadirachta indica* (neem), *Backhousia* spp., *Bambusa* spp. (bamboo), *Beta vulgaris* (sugar beet), *Boehmeria nivea* (ramie), *bok choy, Boronia megastigma* (sweet boronia), *Brassica carinata* (Abyssinian mustard), *Brassica juncea* (Indian mustard), *Brassica napus* (rapeseed), *Brassica oleracea* (cabbage, broccoli), *Brassica oleracea* var Albogabra (gai lum), *Brassica parachinensis* (choi sum), *Brassica pekensis* (Wong bok or Chinese cabbage), *Brassica* spp., *Burrella obovata, Cajanus cajan* (pigeon pea), *Camellia sinensis* (tea), *Cannabis sativa* (non-drug hemp), *Capsicum* spp., *Carica* spp. (papaya); *Carthamus tinctorius* (safflower), *Carum carvi* (caraway), *Cassinia* spp., *Castanospermum australe* (blackbean), *Casuarina cunninghamiana* (beefwood), *Ceratonia siliqua* (carob), *Chamaemelum nobile* (chamomile), *Chamelaucium* spp. (Geraldton wax), *Chenopodium quinoa* (quinoa), *Chrysanthemum* (Tanacetum), *cinerariifolium* (pyrethrum), *Cicer arietinum* (chickpea), *Cichorium intybus* (chicory), *Clematis* spp., *Clianthus formosus* (Sturt's desert pea), *Cocos nucifera* (coconut), *Coffea* spp. (coffee), *Colocasia esculenta* (taro), *Coriandrum sativum* (coriander), *Crambe abyssinica* (crambe), *Crocus sativus* (saffron), *Cucurbita foetidissima* (buffalo gourd), *Cucurbita* spp. (gourd), *Cyamopsis tetragonoloba* (guar), *Cymbopogon* spp. (lemongrass), *Cytisus proliferus* (tagasaste), *Daucus carota* (carrot), *Desmanthus* spp., *Dioscorea esculenta* (Asiatic yam), *Dioscorea* spp. (yams), *Diospyros* spp. (persimmon), *Doronicum* sp., *Echinacea* spp., *Eleocharis dulcis* (water chestnut), *Eleusine coracana* (finger millet), *Emanthus arundinaceus, Eragrostis tef* (tef), *Erianthus jarundinaceus, Eriobonya japonica* (loquat), *Eucalyptus* spp., *Eucalyptus* spp. (gil mallee), *Euclea* spp., *Eugenia malaccensis* (jumba), *Euphorbia* spp., *Euphoria longana* (longan), *Eutrema wasabi* (wasabi), *Fagopyrum esculentum* (buckwheat), *Festuca arundinacea* (tall fescue), *Ficus* spp. (fig), *Flacourtia inermis, Flindersia grayliana* (Queensland maple), *Foeniculum olearia, Foeniculum vulgare* (fennel), *Garcinia mangostana* (mangosteen), *Glycine latifolia, Glycine max* (soybean), *Glycine max* (vegetable soybean), *Glycyrrhiza glabra* (licorice), *Gossypium* spp. (cottons), *Grevillea* spp., *Grindelia* spp., *Guizotia abyssinica* (niger), *Harpagophyllum* sp., *Helianthus annuus* (high oleic sunflowers), *Helianthus annuus* (monosun sunflowers), *Helianthus tuberosus* (Jerusalem artichoke), *Hibiscus cannabinus* (kenaf), *Hordeum bulbosum, Hordeum* spp. (waxy barley), *Hordeum vulgare* (barley), *Hordeum vulgare* subsp. spontaneum, *Humulus lupulus* (hops), *Hydrastis canadensis* (golden seal), *Hymenachne* spp., *Hyssopus officinalis* (hyssop), *Indigofera* spp., *Inga edulis* (ice cream bean), *Inocarpus tugiter, Ipomoea batatas* (sweet potato), *Ipomoea* sp. (kang kong), *Lablab purpureus* (white lablab), *Lactuca* spp. (lettuce), *Lathyrus* spp. (vetch), *Lavandula* spp. (lavender), *Lens* spp. (lentil), *Lesquerella* spp. (bladderpod), *Leucaena* spp., *Lilium* spp., *Limnanthes* spp. (meadowfoam), *Linum usitatissimum* (flax), *Linum usitatissimum* (linseed), *Linum usitatissimum* (Linola.TM.), *Litchi chinensis* (lychee), *Lotus corniculatus* (birdsfoot trefoil), *Lotus pedunculatus,* Lotus sp., Luffa spp., *Lunaria annua* (honesty), *Lupinus mutabilis* (pearl lupin), *Lupinus* spp. (lupin), *Macadamia* spp., *Mangifera indica* (mango), *Manihot esculenta* (cassava), *Medicago* spp. (lucerne), *Medicago* spp., *Melaleuca* spp. (tea tree), *Melaleuca uncinata* (broombush), *Mentha tasmannia, Mentha spicata* (spearmint), *Mentha X piperita* (peppermint), *Momordica charantia* (bitter melon), Musa spp. (banana), *Myrciaria cauliflora* (jaboticaba), *Myrothamnus flabellifolia, Nephelium lappaceum* (rambutan), *Nerine spp., Ocimum basilicum* (basil), *Oenanthe javanica* (water dropwort), *Oenothera biennis* (evening primrose), *Olea europaea* (olive), *Olearia* sp., *Origanum* spp. (marjoram, oregano), *Oryza* spp. (rice), *Oxalis tuberosa* (oca), *Ozothamnus* spp. (rice flower), *Pachyrrhizus ahipa* (yam bean), *Panax* spp. (ginseng), *Panicum miliaceum* (common millet), *Papaver* spp. (poppy), *Parthenium argentatum* (guayule), *Passiflora* sp., *Paulownia tomemtosa* (princess tree), *Pelargonium graveolens* (rose geranium), *Pelargonium* sp., *Pennisetum americanum* (bulrush or pearl millet), *Persoonia* spp., *Petroselinum crispum* (parsley), *Phacelia tanacetifolia* (tansy), *Phalaris canariensis* (canary grass), *Phalaris* sp., *Phaseolus coccineus* (scarlet runner bean), *Phaseolus* lunatus (lima bean), *Phaseolus* spp., *Phaseolus vulgaris* (culinary bean), *Phaseolus vulgaris* (navy bean), *Phaseolus vulgaris* (red kidney bean), *Pisum sativum* (field pea), *Plantago ovata* (psyllium), *Polygonum minus, Polygonum odoratum, Prunus mume* (Japanese apricot), *Psidium guajava* (guava), *Psophocarpus tetragonolobus* (winged bean), *Pyrus* spp. (nashi), *Raphanus satulus* (long white radish or Daikon), *Rhagodia* spp. (saltbush), *Ribes nigrum* (black currant), *Ricinus communis* (castor bean), *Rosmarinus officinalis* (rosemary), *Rungia klossii* (rungia), *Saccharum officinarum* (sugar cane), *Salvia offcinalis* (sage), *Salvia sclarea* (clary sage), *Salvia* sp., *Sandersonia* sp., *Santalum acuminatum* (sweet quandong), *Santalum* spp. (sandalwood), *Sclerocarya caffra* (marula), *Scutellaria galericulata* (scullcap), *Scale cereale* (rye), *Sesamum indicum* (sesame), *Setaria italica* (foxtail millet), *Simmondsia* spp. (jojoba), *Solanum* spp., *Sorghum almum* (sorghum), *Stachys betonica* (wood betony), *Stenanthemum scortechenii, Strychnos cocculoides* (monkey orange), *Stylosanthes* spp. (stylo), *Syzygium* spp., *Tasmannia lanceolata* (mountain pepper), *Terminalia kambachii, Theobroma cacao* (cocoa), *Thymus vulgaris* (thyme), *Toona australis* (red cedar), *Trifoliium* spp. (clovers), *Trifolium alexandrinum* (berseem clover), *Triforium resupinatum* (persian clover), *Triticum* spp., *Triticum tauschii, Tylosema esculentum* (morama bean), *Valeriana* sp. (valerian), *Vernonia* spp., *Vetiver zizanioides* (vetiver grass), *Vicia benghalensis* (purple vetch), *Vicia faba* (faba bean), *Vicia narbonensis* (narbon bean), *Vicia sativa, Vicia* spp., *Vigna aconitifolia* (mothbean), *Vigna angularis* (adzuki bean), *Vigna mungo* (black gram), *Vigna radiata* (mung bean), *Vigna* spp., *Vigna unguiculata* (cowpea), *Vitis* spp. (grapes), *Voandzeia subterranea* (bambarra groundnut), *Triticosecale* (triticale), *Zea mays* (bicolour sweetcorn), *Zea mays* (maize), *Zea mays* (sweet corn), *Zea mays* subsp. mexicana (teosinte), *Zieria* spp., *Zingiber officinale* (ginger), *Zizania* spp. (wild rice), *Ziziphus jujuba* (common jujube). Desirable crops for the practice of the present invention include *Nicotiana tabacum* (tobacco) and horticultural crops such as, for example, *Ananas comosus* (pineapple), *Saccharum* spp (sugar cane), *Musa* spp (banana), Lycopersicon *esculentum* (tomato) and *Solanum tuberosum* (potato).

The synthetic constructs of the invention may be introduced directly into an organism of interest or into one or more of its parts, e.g., cell or tissue types (e.g., a muscle, skin, brain, lung, kidney, pancreas, a reproductive organ such as testes, ovaries and breast, eye, liver, heart, vascular cell, root, leaf, flower, stalk or meristem) or into an organ of the organism. Alternatively, the synthetic construct is introduced into a progenitor of the organism and the progenitor is then grown or cultured for a time and under conditions sufficient to produce the organism of interest, whereby the synthetic construct is contained in one or more cell types of that organism. Suitable progenitor cells include, but are not limited to, stem cells such as non-human embryonic stem cell, pluripotential immune cells, meristematic cells and embryonic callus. In certain embodiments, the synthetic construct is introduced into the organism of interest using a particular route of administration (e.g, for mammals, by the oral, parenteral (e.g., intravenous, intramuscular, intraperitoneal, intaventricular, intrarticular), mucosal (e.g., intranasal, intrapulmonary, oral, buccal, sublingual, rectal, intravaginal), dermal (topical, subcutaneous, transdermal); for plants, administration to flowers, meristem, root, leaves or stalk). Practitioners in the art will recognise that the route of administration will differ depending on the choice of organism of interest and the sought-after phenotype. Desirably, the synthetic constructs are introduced into the same or corresponding site. In other embodiments, the synthetic construct is introduced into a cell of the organism of interest (e.g., autologous cells), or into a cell that is compatible with the organism of interest (e.g., syngeneic or allogeneic cells) and the genetically-modified cell so produced is introduced into the organism of interest at a selected site or into a part of that organism.

The synthetic constructs of the invention may be introduced into an organism of interest or part thereof using any suitable method, and the kind of method employed will differ depending on the intended cell type, part and/or organism of interest. For example, four general classes of methods for delivering nucleic acid molecules into cells have been described: (1) chemical methods such as calcium phosphate precipitation, polyethylene glycol (PEG)-mediate precipitation and lipofection; (2) physical methods such as microinjection, electroporation, acceleration methods and vacuum infiltration; (3) vector based methods such as bacterial and viral vector-mediated transformation; and (4) receptor-mediated. Transformation techniques that fall within these and other classes are well known to workers in the art, and new techniques are continually becoming known. The particular choice of a transformation technology will be determined by its efficiency to transform certain host species as well as the experience and preference of the person practising the invention with a particular methodology of choice. It will be apparent to the skilled person that the particular choice of a transformation system to introduce a synthetic construct of the invention into cells is not essential to or a limitation of the invention, provided it achieves an acceptable level of nucleic acid transfer. Thus, the synthetic constructs are introduced into tissues or host cells by any number of routes, including viral infection, phage infection, microinjection, electroporation, or fusion of vesicles, lipofection, infection by *Agrobacterium tumefaciens* or *A. rhizogenes,* or protoplast fusion. Jet injection may also be used for intra-muscular administration (as described for example by Furth et al., 1992, Anal Biochem 205:365-368). The synthetic constructs may be coated onto microprojectiles, and delivered into a host cell or into tissue by a particle bombardment device, or "gene gun" (see, for example, Tang et al., 1992, Nature 356:152-154). Alternatively, the synthetic constructs can be fed directly to, or injected into, the host organism or it may be introduced into the cell (i.e., intracellularly) or introduced extracellularly into a cavity, interstitial space, into the circulation of an organism, introduced orally, etc. Methods for oral introduction include direct mixing of the synthetic constructs with food of the organism. In certain embodiments, a hydrodynamic nucleic acid administration protocol is employed (e.g., see Chang et al., 2001, J. Virol. 75:3469-3473; Liu et al., 1999, Gene Ther. 6:1258-1266; Wolff et al., 1990, Science 247:1465-1468; Zhang et al., 1999, Hum. Gene Ther. 10:1735-1737; and Zhang et al., 1999, Gene Ther. 7:1344-1349). Other methods of nucleic acid delivery include, but are not limited to, liposome-mediated transfer, naked DNA delivery (direct injection) and receptor-mediated transfer (ligand-DNA complex).

In accordance with the present invention, the individual synthetic constructs are separately introduced into test organisms which are preferably selected from organisms of the same species as the organism of interest or organisms that are related to the organism of interest, or into test parts of such organisms. Related organisms are generally species within the same phylum, preferably species within the same subphylum, more preferably species within superclass, even more preferably species within the same class, even more preferably species within the same order and still even more preferably species within the same genus. For example, if the organism of interest is human, a related species is suitably selected from mouse, cow, dog or cat, which belong to the same class as human, or a chimpanzee, which belongs to the same order as human. Alternatively, if the organism of interest is banana, the related organism may be selected from taro, ginger, onions, garlic, pineapple, bromeliaeds, palms, orchids, lilies, irises and the like, which are all non-graminaceous monocotyledonous plants and which constitute horticultural or botanical relatives.

After introduction of the synthetic constructs into the test organisms or parts, the qualities of their phenotypes are determined by a suitable assay and then compared to determine the relative phenotypic preferences of the synonymous codons. The quality is suitably a measure of the strength, intensity or grade of the phenotype, or the relative strength, intensity or grade of two or more desired phenotypic traits.

Assays for various phenotypes conferred by the production of a chosen reporter protein are known by those of skill in the art. For example, immunity may be assayed by any suitable methods that detects an increase in an animal's capacity to respond to foreign or disease-specific antigens (e.g., cancer antigens) i.e., those cells primed to attack such antigens are increased in number, activity, and ability to detect and destroy the those antigens. Strength of immune response is measured by standard tests including: direct measurement of peripheral blood lymphocytes by means known to the art; natural killer cell cytotoxicity assays (see, e.g., Provinciali et al (1992, J. Immunol. Meth. 155: 19-24), cell proliferation assays (see, e.g., Vollenweider and Groseurth (1992, J. Immunol. Meth. 149: 133-135), immunoassays of immune cells and subsets (see, e.g., Loeffler et al. (1992, Cytom. 13: 169-174); Rivoltini et al. (1992, Can. Immunol. Immunother. 34: 241-251); or skin tests for cell-mediated immunity (see, e.g., Chang et al (1993, Cancer Res. 53: 1043-1050). Enhanced immune response is also indicated by physical manifestations such as fever and inflammation, as well as healing of systemic and local infections, and reduction of symptoms in disease, i.e., decrease in tumour size, alleviation of symptoms of a disease or condition including, but not restricted to, leprosy, tuberculosis, malaria, naphthous ulcers, herpetic and papillomatous warts, gingivitis, artherosclerosis, the concomitants of AIDS such as Kaposi's sarcoma, bronchial infections, and the like. Such physical manifestations may also be used to detect, or define the quality of, the phenotype or class of phenotype displayed by an organism. Alternatively, herbicide tolerance may be assayed by treating test organisms (e.g., plants such as cotton plants), which express a herbicide tolerance gene (e.g., glyphosate tolerance protein gene such as a glyphosate resistant EPSP synthase), with a herbicide (e.g., glyphosate) and determining the efficacy of herbicide tolerance displayed by the plants. For example, when determining the efficacy of synthetic constructs for conferring herbicide tolerance in cotton, the amount of boll retention is a measure of efficacy and is a desirable trait.

The qualities of selected phenotype displayed by the test organisms or by the test parts are then compared to provide a ranked order of the individual codons, encoding a specific amino acid, that are tandemly repeated in the synthetic constructs according to their preference of usage by the organism or part to confer the selected phenotype. One of ordinary skill in the art will thereby be able to determine a "codon preference table" for each amino acid in the polypeptide whose expression conveys the selected phenotype to the organism of interest. Comparison of synonymous codons within a codon preference table can then be used to identify codons for tailoring a synthetic polynucleotide to modulate the quality of a selected phenotype.

### 3. Selection of synonymous and first codons

In accordance with the present invention, a comparison of phenotypic preferences can be determined for synonymous codons in an organism of interest or in a related organism, or in test parts thereof, which can be used as a basis for constructing a synthetic polynucleotide from which a phenotype-associated polypeptide is producible in the organism of interest or part thereof. The synthetic polynucleotide is constructed so that its expression in the organism or part confers a selected phenotype upon that organism or part but in a different quality than that conferred by a parent polynucleotide that encodes the same polypeptide. The method comprises selecting a first codon of the parent polynucleotide for replacement with a synonymous codon, wherein the synonymous codon is selected on the basis that it exhibits a different phenotypic preference than the first codon in a comparison of phenotypic preferences in the organism of interest or in a related organism, or in a part thereof, as determined in Section 2. The first codon is then replaced with the synonymous codon to construct the synthetic polynucleotide.

Thus, in accordance with the present invention, a parent polynucleotide can be modified with synonymous codons such that quality of the selected phenotype conferred by the polynucleotide so modified (synthetic polynucleotide) is higher than from the parent polynucleotide. Generally, the difference between the respective phenotypic qualities conferred by a synthetic polynucleotide and by a parent polynucleotide depends on the number of first codons that are replaced by synonymous codons, and on the difference in phenotypic preference between the first codons and the synonymous codons in the organism of interest or part thereof. Put another way, the fewer such replacements, and/or the smaller the difference in phenotypic preference between the synonymous and first codons, the smaller the difference will be in the phenotypic quality between the synthetic and parent polynucleotides. Conversely, the more such replacements, and/or the greater the difference in phenotypic preference between the synonymous and first codons, the greater the difference will be in the phenotypic quality between the synthetic and parent polynucleotides.

In one embodiment, the present invention contemplates a method of constructing a synthetic polynucleotide that encodes a phenotype-associated polypeptide and that confers a higher quality of a selected phenotype displayed by an organism of interest or part thereof than the quality conferred by a parent polynucleotide that codes for the same polypeptide. In this embodiment, a first codon of the parent polynucleotide is selected for replacement with a synonymous codon,
wherein the synonymous codon is selected on the basis that it exhibits a higher phenotypic preference than the first codon in a comparison of phenotypic preferences in the organism of interest or in a related organism, or in a part thereof.

In accordance with the present invention, a higher quality of a selected phenotype can be achieved by selecting a synonymous codon that has a higher phenotypic preference than the first codon. Generally, a higher phenotypic preference will correlate with a higher quality of the selected phenotype. Thus, in a non-limiting example of such a correlation, a synonymous codon is deemed to have at least about a 5% higher phenotypic preference than a first codon when the quality of phenotype displayed by an organism or part thereof to which a synthetic construct comprising a tandem repeat of the synonymous codon has been provided is at least about 5% higher than the quality of phenotype displayed by an organism or part thereof to which a synthetic construct comprising a tandem repeat of the first codon has been provided. When selecting the synonymous codon, it is preferred that it has a phenotypic preference in the organism of interest that is at least about 105%, suitably at least about 110%, preferably at least about 120%, more preferably at least about 130%, even more preferably at least about 140%, even more preferably at least about 150%, even more preferably at least about 160%, even more preferably at least about 170%, even more preferably at least about 180%, even more preferably at least about 190%, even more preferably at least about 200%, even more preferably at least about 250%, even more preferably at least about 300%, even more preferably at least about 350%, even more preferably at least about 400%, even more preferably at least about 450%, even more preferably at least about 500%, even more preferably at least about 550%, even more preferably at least about 600%, even more preferably at least about 650%, and still even more preferably at least about 700% of the phenotypic preference of the first codon. In the case of two or more synonymous codons having similar phenotypic preferences, it will be appreciated that any one of these codons can be used to replace the first codon. Generally, if a parent polynucleotide has a choice of low and intermediate phenotypic preference codons, it is preferable in the first instance to replace some, or more preferably all, of the low phenotypic preference codons with synonymous codons having intermediate, or preferably high, phenotypic preferences. Typically, replacement of low with intermediate or high phenotypic preference codons results in a substantial increase in the quality of the phenotype conferred by the synthetic polynucleotide so constructed. However, it is also preferable to replace some, or preferably all, of the intermediate phenotypic preference codons with high translationally efficient codons for conferring an optimal quality in the selected phenotype.

In another embodiment, the present invention contemplates a method of constructing a synthetic polynucleotide that encodes a phenotype-associated polypeptide and that confers a lower quality of a selected phenotype displayed by an organism of interest or part thereof than the quality conferred by a parent polynucleotide that codes for the same polypeptide. In this embodiment, a first codon of the parent polynucleotide is selected for replacement with a synonymous codon, wherein the synonymous codon is selected on the basis that it exhibits a lower phenotypic preference than the first codon in a comparison of phenotypic preferences in the organism of interest or in a related organism or in a part thereof.

In accordance with the present invention, a lower quality of a selected phenotype can be achieved by selecting a synonymous codon that has a lower phenotypic preference than the first codon. A lower phenotypic preference will typically correlate with a lower quality of the selected phenotype. Accordingly, in a non-limiting example of such a correlation, a synonymous codon is deemed to have at least about a 5% lower phenotypic preference than a first codon when the quality of phenotype displayed by an organism or part thereof to which a synthetic construct comprising a tandem repeat of the synonymous codon has been provided is at least about 5% lower than the quality of phenotype displayed by an organism or part thereof to which a synthetic construct comprising a tandem repeat of the first codon has been provided. When selecting the synonymous codon for this embodiment, it is preferred that it has a phenotypic preference in the organism of interest that is at least about 95%, suitably at least about 90%, preferably at least about 85%, more preferably at least about 80%, even more preferably at least about 75%, even more preferably at least about 70%, even more preferably at least about 65%, even more preferably at least about 60%, even more preferably at least about 55%, even more preferably at least about 50%, even more preferably at least about 45%, even more preferably at least about 40%, even more preferably at least about 35%, even more preferably at least about 30%, even more preferably at least about 25%, even more preferably at least about 20%, even more preferably at least about 15%, even more preferably at least about 10%, and still even more preferably at least about 5% of the phenotypic preference of the first codon.

It is preferable but not necessary to replace all the codons of the parent polynucleotide with synonymous codons having higher or lower phenotypic preference in the organism of interest or part thereof than the first codons. For example, a higher or lower phenotypic quality can be accomplished even with partial replacement. Typically, the replacement step affects 5%, 10%, 15%, 20%, 25%, 30%, more preferably 35%, 40%, 50%, 60%, 70% or more of the first codons of the parent polynucleotide. In a preferred embodiment requiring a higher phenotypic quality, the number of, and difference in phenotypic preference between, the first codons and the synonymous codons are selected such that the phenotype-associated polypeptide is produced from the synthetic polynucleotide to confer a phenotype upon a chosen organism or organism part in a quality that is at least about 110%, suitably at least about 150%, preferably at least about 200%, more preferably at least about 250%, even more preferably at least about 300%, even more preferably at least about 350%, even more preferably at least about 400%, even more preferably at least about 450%, even more preferably at least about 500%, and still even more preferably at least about 1000%, of the quality of phenotype conferred by the parent polynucleotide in the organism or part. Conversely, in a preferred embodiment requiring a lower phenotypic quality, the number of, and difference in phenotypic preference between, the first codons and the synonymous codons are selected such that the phenotype-associated polypeptide is produced from the synthetic polynucleotide to confer a phenotype upon a chosen organism or part thereof in a quality that is no more than about 90%, suitably no more than about 80%, preferably no more than about 70%, more preferably no more than about 60%, even more preferably no more than about 50%, even more preferably no more than about 40%, even more preferably no more than about 30%, even more preferably no more than about 20%, even more preferably no more than about 10%, and still even more preferably no more than about 5%, of the quality of phenotype conferred by the parent polynucleotide in the organism or part.

### 4. Construction of synthetic polynucleotides

Replacement of one codon for another can be achieved using standard methods known in the art. For example codon modification of a parent polynucleotide can be effected using several known mutagenesis techniques including, for example, oligonucleotide-directed mutagenesis, mutagenesis with degenerate oligonucleotides, and region-specific mutagenesis. Exemplary *in vitro* mutagenesis techniques are described for example in U.S. Patent Nos. 4,184,917, 4,321,365 and 4,351,901 or in the relevant sections of Ausubel, et al. (CURRENT PROTOCOLS IN MOLECULAR BIOLOGY, John Wiley & Sons, Inc. 1997) and of Sambrook, et al., (MOLECULAR CLONING. A LABORATORY MANUAL, Cold Spring Harbor Press, 1989). Instead of *in vitro* mutagenesis, the synthetic polynucleotide can be synthesised *de novo* using readily available machinery as described, for example, in U.S. Patent No 4,293,652. However, it should be noted that the present invention is not dependent on, and not directed to, any one particular technique for constructing the synthetic polynucleotide.

The parent polynucleotide is suitably a natural gene. However, it is possible that the parent polynucleotide that is not naturally-occurring but has been engineered using recombinant techniques. Parent polynucleotides can be obtained from any suitable source, such as from eukaryotic or prokaryotic organisms, including but not limited to mammals or other animals, and pathogenic organisms such as yeasts, bacteria, protozoa and viruses.

The invention also contemplates synthetic polynucleotides encoding one or more desired portions of the phenotype-associated polypeptide. In this regard, it is preferable that the synthetic polynucleotide encodes at least one functional domain of the phenotype-associated polypeptide, which is preferably at least about 10, more preferably at least about 20, even more preferably at least about 50, even more preferably at least about 100, even more preferably at least about 150, and still more preferably at least about 500 contiguous amino acid residues of that polypeptide.

The invention further contemplates a synthetic construct (or expression vector), comprising a synthetic polynucleotide of the invention, which is operably linked to a regulatory polynucleotide. The regulatory polynucleotide suitably comprises transcriptional and/or translational control sequences, which will be compatible for expression in the organism of interest or in cells of that organism. Typically, the transcriptional and translational regulatory control sequences include, but are not limited to, a promoter sequence, a 5' non-coding region, a *cis-*regulatory region such as a functional binding site for transcriptional regulatory protein or translational regulatory protein, an upstream open reading frame, ribosomal-binding sequences, transcriptional start site, translational start site, and/or nucleotide sequence which encodes a leader sequence, termination codon, translational stop site and a 3' non-translated region. Constitutive or inducible promoters as known in the art are contemplated by the invention. The promoters may be either naturally occurring promoters, or hybrid promoters that combine elements of more than one promoter. Promoter sequences contemplated by the present invention may be native to the organism of interest or may be derived from an alternative source, where the region is functional in the chosen organism. The choice of promoter will differ depending on the intended host. For example, promoters which could be used for expression in plants include plant promoters such as: constitutive plant promoters examples of which include CaMV35S plant promoter, CaMV19S plant promoter, FMV34S plant promoter, sugarcane bacilliform badnavirus plant promoter, CsVMV plant promoter, *Arabidopsis* ACT2/ACT8 actin plant promoter, Arabidopsis ubiquitin UBQ1 plant promoter, barley leaf thionin BTH6 plant promoter, and rice actin plant promoter; tissue specific plant promoters examples of which include bean phaseolin storage protein plant promoter, DLEC plant promoter, PHS.beta. plant promoter, zein storage protein plant promoter, conglutin gamma plant promoter from soybean, AT2S1 gene plant promoter, ACT11 actin plant promoter from *Arabidopsis, napA* plant promoter from *Brassica napus* and potato patatin gene plant promoter; and inducible plant promoters examples of which include a light-inducible plant promoter derived from the pea *rbcs* gene, a plant promoter from the alfalfa *rbcS* gene, DRE, MYC and MYB plant promoters which are active in drought; INT, INPS, *prxEa,* Ha hsp17.7G4 and RD21 plant promoters active in high salinity and osmotic stress, and hsr203J and str246C plant promoters active in pathogenic stress. Alternatively, promoters which could be used for expression in mammals include the metallothionein promoter, which can be induced in response to heavy metals such as cadmium, the β-actin promoter as well as viral promoters such as the SV40 large T antigen promoter, human cytomegalovirus (CMV) immediate early (IE) promoter, rous sarcoma virus LTR promoter, adenovirus promoter, or a HPV promoter, particularly the HPV upstream regulatory region (URR) may also be used. All these promoters are well described and readily available in the art.

The synthetic construct of the present invention may also comprise a 3' non-translated sequence. A 3' non-translated sequence refers to that portion of a gene comprising a DNA segment that contains a polyadenylation signal and any other regulatory signals capable of effecting mRNA processing or gene expression. The polyadenylation signal is characterised by effecting the addition of polyadenylic acid tracts to the 3' end of the mRNA precursor. Polyadenylation signals are commonly recognised by the presence of homology to the canonical form 5' AATAAA-3' although variations are not uncommon. The 3' non-translated regulatory DNA sequence preferably includes from about 50 to 1,000 nucleotide base pairs and may contain transcriptional and translational termination sequences in addition to a polyadenylation signal and any other regulatory signals capable of effecting mRNA processing or gene expression.

In a preferred embodiment, the synthetic construct further contains a selectable marker gene to permit selection of an organism or a precursor thereof that contains the synthetic construct. Selection genes are well known in the art and will be compatible for expression in organism or its precursor.

It will be understood, however, that expression of protein-encoding polynucleotides in heterologous systems is now well known, and the present invention is not directed to or dependent on any particular vector, transcriptional control sequence or technique for its production. Rather, synthetic polynucleotides prepared according to the methods set forth herein may be introduced into an organism of interest or into a precursor or progenitor thereof in any suitable manner in conjunction with any suitable synthetic construct or vector, and the synthetic polynucleotides may be expressed with known promoters in any conventional manner.

In some embodiments, the synthetic constructs of the invention are in the form of viral vectors, such as simian virus 40 (SV40) or bovine papilloma virus (BPV), which has the ability to replicate as extra-chromosomal elements (Eukaryotic Viral Vectors, Cold Spring Harbor Laboratory, Gluzman ed., 1982; Sarver et al., 1981, Mol. Cell. Biol. 1:486). Viral vectors include retroviral (lentivirus), adeno-associated virus (see, e.g., Okada, 1996, Gene Ther. 3:957-964; Muzyczka, 1994, J. Clin. Invst. 94:1351; U.S. Pat. Nos. 6,156,303; 6,143,548 5,952,221, describing AAV vectors; see also U.S. Pat. Nos. 6,004,799; 5,833,993), adenovirus (see, e.g., U.S. Pat. Nos. 6,140,087; 6,136,594; 6,133,028; 6,120,764), reovirus, herpesvirus, rotavirus genomes etc., modified for introducing and directing expression of a polynucleotide or transgene in cells. Retroviral vectors can include those based upon murine leukemia virus (see, e.g., U.S. Pat. No. 6,132,731), gibbon ape leukemia virus (see, e.g., U.S. Pat. No. 6,033,905), simian immuno-deficiency virus, human immuno-deficiency virus (see, e.g., U.S. Pat. No. 5,985,641), and combinations thereof.

Vectors also include those that efficiently deliver genes to animal cells *in vivo* (e.g., stem cells) (see, e.g., U.S. Pat. Nos. 5,821,235 and 5,786,340; Croyle et al., 1998, Gene Ther. 5:645; Croyle et al., 1998, Pharm. Res. 15:1348; Croyle et al., 1998, Hum. Gene Ther. 9:561; Foreman et al., 1998, Hum. Gene Ther. 9:1313 ; Wirtz et al., 1999, Gut 44:800). Adenoviral and adeno-associated viral vectors suitable for *in vivo* delivery are described, for example, in U.S. Pat. Nos. 5,700,470, 5,731,172 and 5,604,090. Additional vectors suitable for *in vivo* delivery include herpes simplex virus vectors (see, e.g., U.S. Pat. No. 5,501,979), retroviral vectors (see, e.g., U.S. Pat. Nos. 5,624,820, 5,693,508 and 5,674,703; and WO92/0526 and WO92/14829), bovine papilloma virus (BPV) vectors (see, e.g., U.S. Pat. No. 5,719,054), CMV-based vectors (see, e.g., U.S. Pat. No. 5,561,063) and parvovirus, rotavirus and Norwalk virus vectors. Lentiviral vectors are useful for infecting dividing as well as non-dividing cells (see, e.g., U.S. Pat. No. 6,013,516).

Vectors for insect cell expression commonly use recombinant variations of baculoviruses and other nucleopolyhedrovirus, e.g., Bombyx mori nucleopolyhedrovirus vectors (see, e.g., Choi, 2000, Arch. Virol. 145:171-177). For example, Lepidopteran and Coleopteran cells are used to replicate baculoviruses to promote expression of foreign genes carried by baculoviruses, e.g., Spodoptera frugiperda cells are infected with recombinant Autographa californica nuclear polyhedrosis viruses (AcNPV) carrying a heterologous, e.g., a human, coding sequence (see, e.g., Lee, 2000, J. Virol. 74:11873-11880; Wu, 2000, J. Biotechnol. 80:75-83). See, e.g., U.S. Pat. No. 6,143,565, describing use of the polydnavirus of the parasitic wasp Glyptapanteles indiensis to stably integrate nucleic acid into the genome of Lepidopteran and Coleopteran insect cell lines. See also, U.S. Pat. Nos. 6,130,074; 5,858,353; 5,004,687.

Expression vectors capable of expressing proteins in plants are well known in the art, and include, e.g., vectors from Agrobacterium spp., potato virus X (see, e.g., Angell, 1997, EMBO J. 16:3675-3684), tobacco mosaic virus (see, e.g., Casper, 1996, Gene 173:69-73), tomato bushy stunt virus (see, e.g., Hillman, 1989, Virology 169:42-50), tobacco etch virus (see, e.g., Dolja, 1997, Virology 234:243-252), bean golden mosaic virus (see, e.g., Morinaga, 1993, Microbial Immunol. 37:471-476), cauliflower mosaic virus (see, e.g., Cecchini, 1997, Mol. Plant Microbe Interact. 10:1094-1101), maize Ac/Ds transposable element (see, e.g., Rubin, 1997, Mol. Cell. Biol, 17:6294-6302; Kunze, 1996, Curr. Top. Microbiol. Immunol. 204:161-194), and the maize suppressor-mutator (Spm) transposable element (see, e.g., Schlappi, 1996, Plant Mol. Biol. 32:717-725); and derivatives thereof.

The invention further contemplates organisms containing therein the synthetic construct of the invention, or alternatively, parts, precursors, cells or tissues produced by the methods of the invention.

In order that the invention may be readily understood and put into practical effect, particular preferred embodiments will now be described by way of the following non-limiting examples.

### EXAMPLES

### EXAMPLE 1

### Codon-modification of an antigen-encoding polynucleotide to enhance the antibody response of a mammal to that antigen

### Reagents:

For each set of synonymous codons that code for a single amino acid, a set of 2-6 gene constructs is constructed, each encoding green fluorescent protein with a leader sequence comprising six copies of an amino acid. Each member of the set is identical except that, across the set, the leader sequence comprises 6 copies of each of the possible synonymous codon triplets (XXX) that code for the amino acid.

The general format of these gene constructs would be:

Eukaryotic Promoter - ATG - (XXX)6 - GFP gene - polyadenylation signal

The nucleic acid and deduced amino acids sequences of the GFP gene are set forth in SEQ ID NO: 1 and 2, respectively. The nucleic acid and deduced amino acids sequences of the different leader sequences are set forth in SEQ ID NO: 3 to 120.

A superset of 20 such sets of gene constructs is thus constructed, encoding all possible naturally occurring amino acids for which there are redundant coding triplets, comprising in total 59 gene constructs.

### Method:

Groups of 8-10 6 wk old Balb/c female mice are immunised transcutaneously using a biolistic delivery system (e.g., the Biorad gene gun or the Powderject delivery system) with one of the 59 gene constructs, on one to three occasions at three weekly intervals. Plasmid (1-2 µg) are coated onto the gold beads (Biorad) or sugar (Powderject) material according to the manufacturers instructions. Beads are delivered to the shaven abdominal skin of the mouse. The Powderject system is optimised for delivery to the basal epidermis. The Biorad system has the capacity to alter delivery depth by adjusting the gas pressure used for the delivery. Data are collected for gas pressures optimised for delivery to the superficial, or basal epithelium, or to the dermis. This is calibrated for the particular gene gun and beads by delivering beads to skin and examining microscopic sections to score the percentage of beads at various depths in the dermis and epidermis.

Serum samples are taken by retro-orbital bleeding at weeks 3 and 6 post immunisation and by terminal cardiac puncture at week 9 post immunisation and the titre of antibody to GFP determined by ELISA for each serum. For ELISA, GFP (Sigma) is coated onto plates and serum reactivity determined according to standard protocols (see, e.g., Walsh et al., 2000, J Gen Virol. 709-718).

### Outcome and interpretation:

The mean antibody titre to GPF induced by each set of constructs encoding a given amino acid is ranked to determine the optimal codon for antibody production using a polynucleotide delivered by a biolistic delivery system (conversely the optimal codon to avoid antibody production).

A list of optimal codons is thus be produced. An improved gene construct for a polynucleotide vaccine delivered by the tested delivery system to skin and designed to induce maximal antibody titre is one in which codons in the gene of interest (e.g., influenza hemagglutinin) which are not optimal are (in large measure) replaced by ones that are optimal for producing maximal antibody titre.

### EXAMPLE 2

### Codon-modification of an antigen-encoding polynucleotide to confer tolerance to that antigen in a mammal

### Reagents:

For each set of synonymous codons that code for a single amino acid, a set of 2-6 gene constructs is constructed, each encoding glutamic acid decarboxylase (GAD) with a leader sequence comprising six copies of an amino acid. Each member of the set is identical except that, across the set, the leader sequence comprises 6 copies of each of the possible synonymous codon triplets (XXX) that code for the amino acid.

The general format of such gene constructs would be:

Eukaryotic Promoter - ATG - (XXX)6 - GAD gene - polyadenylation signal

The nucleic acid and deduced amino acids sequences of the GAD gene are set forth in SEQ ID NO: 121 and 122, respectively.

A superset of 20 such sets of gene constructs is thus constructed, encoding all possible naturally occurring amino acids for which there are redundant coding triplets, comprising in total 59 gene constructs.

### Methods:

Groups of 4 week old NOD (non obese diabetic) female mice are immunised transcutaneously using a biolistic delivery system (e.g., the Biorad gene gun or the Powderject delivery system) with one of the 59 gene constructs, on one to three occasions at three weekly intervals. Plasmid (1-2 µg) are coated onto the gold beads (Biorad) or sugar (Powderject) material according to the manufacturers instructions. Beads are delivered to the shaven abdominal skin of the mouse. The Powderject system is optimised for delivery to the basal epidermis. The Biorad system has the capacity to alter delivery depth by adjusting the gas pressure used for the delivery. Data are collected for gas pressures optimised for delivery to the superficial, or basal epithelium, or to the dermis. This is calibrated for the particular gene gun and beads by delivering beads to skin and examining microscopic sections to score the percentage of beads at various depths in the dermis and epidermis.

The mice are followed to the onset of diabetes as assessed by a blood sugar level over 10 mM/L on weekly blood samples obtained by retro-orbital bleeding. Onset of diabetes is confirmed by testing for glycosuria, and pancreatic histology determined on the diabetic mice, and on mice deemed free of diabetes at 26 weeks.

### Outcome and interpretation:

The mean time to onset of diabetes for mice immunised with each set of constructs encoding a given amino acid would be ranked, to determine the optimal codon for expressing the gene of interest in the sites responsible for prevention of a pathogenic immune response, using a polynucleotide delivered by a biolistic delivery system.

A list of optimal codons for ensuring protein expression from a polynucleotide vaccine at the optimal sites to induce host protective immunity (tolerance) is thus produced. An improved gene construct for a polynucleotide vaccine delivered by the tested delivery system to skin and designed to induce maximal host protective immune tolerance is one in which codons in the gene of interest (e.g., insulin with a view to controlling human diabetes) which are not optimal are (in large measure) replaced by ones that are optimal.

### EXAMPLE 3

### Codon-modification for optimising the resistance of transgenic plants to herbicides while maintaining seed protein production.

### Reagents:

For each set of synonymous codons that code for a single amino acid, a set of 2-6 gene constructs is constructed, each encoding phosphinothricin acetyltransferase (encoded by the *bar* gene) with a leader sequence comprising six copies of an amino acid. Each member of the set is identical except that, across the set, the leader sequence comprises 6 copies of each of the possible synonymous codon triplets (XXX) that code for the amino acid.

The general format of such gene constructs would be:

Plant Promoter - ATG - (XXX)6 - BAR gene - plant polyadenylation signal

The nucleic acid and deduced amino acids sequences of the BAR gene are set forth in SEQ ID NO: 123 and 124, respectively.

A superset of 20 such sets of gene constructs is thus constructed, encoding all possible naturally occurring amino acids, comprising in total 59 gene constructs.

### Method:

The method of Weeks et al. (1993, Plant Physiol 102(4):1077-1084) for the production of multiple independent lines of fertile transgenic wheat (*Triticum aestivum*)*.* Briefly, calli derived from immature embryos, 0.5 to 1 mm long, are bombarded with microprojectiles coated with DNA containing as marker genes the bar gene, encoding phosphinothricin-resistance, and the gene encoding β-glucuronidase (GUS), each under control of a maize ubiquitin promoter. The bombardment is performed 5 d after embryo excision, just after initiation of callus proliferation. The ability of plantlets to root in the presence of 1 or 3 mg/L of bialaphos is a reliable selection criteria used to identify transformed plants. Stable transformation is confirmed by marker gene expression assays and the presence of the *bar* sequences in high molecular weight chromosomal DNA of the resultant plants. On average, 168 d elapses between embryo excision for bombardment and anthesis of the T0 plants. The transmission of both the resistance phenotype and bar DNA to the T1 generation verified that germline transformation had occurred.

Herbicide resistance is measured using the method of Singh and Wright (2002, Lett. Appl. Microbiol. 35(1):12-16) and protein concentration in seed is measured by the method of Turner et al. (1990, Plant Mol. Biol. 14(5):793-803).

### Outcome and interpretation:

For each amino acid leader sequence, plants are ranked according to their resistance to the Basta Herbicide, and to their seed protein content. The optimal codon from each set is the one which gives plants adequate herbicide resistance and the maximum seed protein yield.

A list of optimal codons is thus be produced, and an improved gene construct for a gene conveying herbicide resistance to a transgenic grain plant is one in which codons in the gene of interest (say those in the broad host range plasmid pJP4, which carries genes for the degradation of 2,4-dichlorophenoxyacetic acid (2,4-D), 2-methyl-4-chlorophenoxyacetic acid, and 3-chlorobenzoic acid (see e.g., Kleinsteuber, et al, 2001, Extremophiles. 5(6):375-384) which are not optimal are (in large measure ) replaced by ones that are optimal.

### EXAMPLE 4

### Codon-modification for optimising the selectivity of a growth inhibitory gene for controlling a skin tumour without damage to surrounding normal skin.

### Reagents:

For each set of synonymous codons that code for a single amino acid, a set of 2-6 gene constructs is constructed, each encoding an E2F1 dominant negative inhibitor with a leader sequence comprising six copies of an amino acid. Each member of the set is identical except that, across the set, the leader sequence comprises 6 copies of each of the possible synonymous codon triplets (XXX) that code for the amino acid.

The general format of such gene constructs would be:

Eukaryotic Promoter - ATG - (XXX)6 - E2Fldn gene - IRES - GFP gene-polyadenylation signal

The nucleic acid and deduced amino acids sequences of the E2F1 dominant negative inhibitor gene are set forth in SEQ ID NO: 125 and 126, respectively.

A superset of 20 such sets of gene constructs is thus constructed, encoding all possible naturally occurring amino acids for which there are redundant coding triplets, comprising in total 59 gene constructs.

### Methods:

Tumours are induced on the skin of FVB mice by painting with chemical carcinogens using a standard DMBA/TPA promotion/induction regime. Groups of tumour bearing female mice are treated topically using a biolistic delivery system (e.g., the Biorad gene gun or the Powderject delivery system) with one of the 59 gene constructs, on one to three occasions at three weekly intervals. Plasmid (1-2 µg) are coated onto the gold beads (Biorad) or sugar (Powderject) material according to the manufacturers instructions. Beads are delivered to the shaven abdominal skin of the mouse. The Powderject system is optimised for delivery to the basal epidermis. The Biorad system has the capacity to alter delivery depth by adjusting the gas pressure used for the delivery. Data are collected for gas pressures optimised for delivery to the superficial, or basal epithelium, or to the dermis. This is calibrated for the particular gene gun and beads by delivering beads to skin and examining microscopic sections to score the percentage of beads at various depths in the dermis and epidermis.

Twenty-four hours after treatment, the skin from the tumour and surrounding normal skin is excised and disaggregated into single cells. Cells are sorted for expression of the GFP by high speed flow cytometry, and the clonogenic potential of the GFP positive cells assessed for each construct, and for normal and tumour tissue. Clonogenic potential is established by plating cells on collagen coated plates in minimal KGM medium, and by counting colonies established per 10000 input cells. The ratio of colonies obtained from normal tissue to colonies obtained from tumour tissue is measure of the efficiency of targeting of that particular construct to the tumour tissue - higher colony forming efficiency for the normal skin cells in comparison to the tumour cells indicates a more preferred codon for correctly delivering gene therapy to the tumour.

### Outcome and interpretation:

The mean colony forming efficiency ratio (normal/tumour cells) are ranked to determine the optimal codon for inhibiting tumour growth while preserving normal skin, using a polynucleotide delivered by a biolistic delivery system. A list of optimal codons is thus produced. An improved gene construct for treating skin cancers delivered by the tested delivery system to skin and designed to induce maximal selectivity for the tumour over normal tissue is one in which codons in the gene of interest for skin cancer treatment (for example p53) most resemble the optimal list.

Throughout the specification the aim has been to describe the preferred embodiments of the invention without limiting the invention to any one embodiment or specific collection of features. Those of skill in the art will therefore appreciate that, in light of the instant disclosure, various modifications and changes can be made in the particular embodiments exemplified without departing from the scope of the present invention. All such modifications and changes are intended to be included within the scope of the appended claims.

## Claims

1. A method for constructing a synthetic polynucleotide from which a polypeptide is producible to confer a selected phenotype upon a multicellular organism of interest or part thereof in a different quality than that conferred by a parent polynucleotide that encodes the same polypeptide, the method comprising: (a) selecting a first codon of the parent polynucleotide for replacement with a synonymous codon, wherein the synonymous codon is selected on the basis that it exhibits a different phenotypic preference than the first codon in a comparison of phenotypic preferences in non-human test organisms or parts thereof, wherein the parts of test organisms are selected from tissues or organs of the test organisms, wherein the test organisms are selected from the group consisting of organisms of the same species as the organism of interest and organism that are related to the organism of interest: and (b) replacing the first codon with the synonymous codon to construct the synthetic polynucleotide, wherein the selected phenotype is other than a phenotype conferred upon a cell by the parent polynucleotide containing the first codon that is expressed in the cell and that encodes the polypeptide.

2. A method according to claim 1, wherein the phenotypic preferences of codons in the test organisms or parts are compared by: (i) separately introducing into the test organisms or parts individual synthetic constructs, each of which comprises a regulatory polynucleotide operably linked to a tandem repeat of a codon fused in frame with a reporter polynucleotide that encodes a reporter protein, which produces, or which is predicted to produce, a corresponding phenotype selected from the group consisting of the selected phenotype and a phenotype of the same class as the selected phenotype; and (ii) comparing the quality of the phenotypes displayed by the test organisms or parts to determine the relative phenotypic preferences of the codons.

3. A method for constructing a synthetic polynucleotide from which a polypeptide is producible to confer a selected phenotype upon a multicellular organism of interest or part thereof in a different quality than that conferred by a parent polynucleotide that encodes the same polypeptide, the method comprising: (a) selecting a first codon of the parent polynucleotide for replacement with a synonymous codon, wherein the synonymous codon is selected on the basis that it exhibits a different phenotypic preference than the first codon in a comparison of phenotypic preferences in ex *vivo* parts of test organisms, wherein said parts of test organisms are selected from tissues or organs of the test organisms, wherein said parts of test organisms do not include a human embryonic stem cell or a human germ line cell, wherein the parts of test organisms are selected from the group consisting of organisms of the same species as the organism of interest and organisms that are related to the organism of interest: and (b) replacing the first codon with the synonymous codon to construct the synthetic polynucleotide, wherein the selected phenotype is other than a phenotype conferred upon a cell by the parent polynucleotide containing the first codon that is expressed in the cell and that encodes the polypeptide.

4. A method according to claim 3, wherein the phenotypic preferences of codons in the parts of test organisms are compared by: (i) separately introducing into the *ex vivo* parts of test organisms individual synthetic constructs, each of which comprises a regulatory polynucleotide operably linked to a tandem repeat of a codon fused in frame with a reporter polynucleotide that encodes a reporter protein, which produces, or which is predicted to produce, a corresponding phenotype selected from the group consisting of the selected phenotype and a phenotype of the same class as the selected phenotype; and (ii) comparing the quality of the phenotypes displayed by the *ex vivo* parts of test organisms to determine the relative phenotypic preferences of the cordons.

5. A method according to claim 2 or 4, wherein the reporter protein produces the selected phenotype.

6. A method according to claim 2 or 4, wherein the reporter does not produce the selected phenotype but produces the same class of phenotype as the selected phenotype.

7. A method according to claim 5 or claim 6, wherein the reporter protein is selected from antigens derived from pathogenic organisms, cancer antigens, self antigens, transplantation antigens, growth factors, hormones and toxins.

8. A method according to any one of claims 1 to 7, wherein the phenotype is selected from immunity, antigen tolerance, angiogenesis, anti-angiogenesis, amelioration of clinical symptoms, reduced or increased cell death, reduced or increased cell differentiation, reduced or increased cell proliferation, tumour or cancer regression, growth and repair of tissue or organ, decreased fibrosis, inhibition or reversal of cell senescence, increased or reduced cell migration, differential expression of protein between different cells or tissues of an organism or part thereof, trauma recovery, recovery from bums, antibiotic resistance or sensitivity, herbicide tolerance or sensitivity, starch biosynthesis or modification, fatty acid biosynthesis, disease resistance or tolerance, pest resistance or tolerance including insect resistance or tolerance, viral resistance or tolerance, fungal resistance or tolerance, a metabolic trait including sucrose metabolism, frost resistance or tolerance, stress tolerance, and improved food content or increased yields.

9. A method according to any one of claims 1 to 7, wherein the phenotype is an immune response.

10. A method according to claim 9, wherein the immune response is a humoral immune response.

11. A method according to claim 9, wherein the immune response is a cell mediated immune response.

12. A method according to claim 9, wherein the immune response is an innate immunity mediated response.

13. A method according to claim 2 or claim 4, wherein the synthetic constructs are introduced into the non-human test organisms using the same or similar mode of introduction.

14. A method according to claim 2 or claim 4, wherein the synthetic constructs are introduced into the non-human test organisms at the same or corresponding site.

15. A method according to claim 1, wherein the organism of interest is a non-human mammal and the synthetic constructs are introduced by oral, intravenous, intramuscular, intranasal, buccal, subcutaneous, transdermal, buccal or sublingual route.

16. A method according to claim 1, wherein the synthetic constructs are introduced into one or more of cell or tissue types of the organism of interest.

17. A method according to claim 16, wherein the synthetic constructs are introduced into cells selected from muscle cells, skin cells, brain cells, lung cells, kidney cells, pancreas cells, cells of a reproductive organ, heart cells, vascular cells, liver cells, eye cells, flower cells, meristematic cells, root cells and leaf cells.

18. A method according to claim 3, wherein the synthetic constructs are introduced into one or more *ex vivo* cell or tissue types of the organism of interest wherein said cell or tissue types do not include a human embryonic stem cell or a human germ line cell.

19. A method according to claim 18, wherein the cells into which the synthetic constructs are introduced into are selected from muscle cells, skin cells, brain cells, lung cells, kidney cells, pancreas cells, cells of a reproductive organ, heart cells, vascular cells, liver cells, eye cells, flower cells, meristematic cells, root cells and leaf cells.

20. A method according to claim 2 or 4, wherein the tandem repeat of each of the synthetic constructs comprises at least three copies of the corresponding codon.

21. A method according to any one of claims 1 to 4, wherein the synonymous codon is selected such that it has a higher phenotypic preference than the first codon.

22. A method according to claim 21, wherein the synonymous codon is selected when the quality of the phenotype conferred by the synthetic construct comprising a tandem repeat of the synonymous codon is at least about 5% higher than the quality of the phenotype conferred by the synthetic construct comprising a tandem repeat of the first codon.

23. A method according to any one of claims 1 to 4, wherein the synonymous codon is selected such that it has a lower phenotypic preference than the first codon.

24. A method according to claim 23, wherein the synonymous codon is selected when the quality of the phenotype conferred by the synthetic construct comprising a tandem repeat of the synonymous codon is at least about 5% lower than the quality of the phenotype conferred by the synthetic construct comprising a tandem repeat of the first codon.

25. A method according to any one of claims 1 to 24, wherein the multicellular organism is an animal.

26. A method according to any one of claims 1 to 24, wherein the multicellular organism is a mammal.

27. A method according to any one of claims 1 to 24, wherein the multicellular organism is a plant.

28. A method according to claim 2, wherein the synthetic constructs are introduced into progenitors of the non-human test organisms or parts and the progenitors are grown or cultured for a time and under conditions sufficient to produce the test organisms or parts, whereby the synthetic constructs are contained in one or more cell types of those organisms or parts.

29. A method according to claim 28, wherein the progenitor cells are selected from stem cells, pluripotential cells, meristematic cells and embryonic callus.

30. A method for determining the phenotypic preference of a first codon in a multicellular organism of interest or part thereof, the method comprising: (a) introducing a synthetic construct into a non-human test organism or part thereof, wherein said part of a test organism is selected from tissues or organs of the test organism, wherein the test organism is selected from the group consisting of an organism of the same species as the organism of interest and an organism that is related to the organism of interest, the synthetic construct comprising a regulatory polynucleotide operably linked to a tandem repeat of the first codon fused in frame with a reporter polynucleotide that encodes a reporter protein, which produces, or which is predicted to produce, a selected phenotype or a phenotype of the same class as the selected phenotype; and (b) determining the quality of the corresponding phenotype displayed by the non-human test organism or part, wherein the selected phenotype or the phenotype of the same class as the selected phenotype is other than a phenotype conferred upon a cell by the parent polynucleotide containing the first codon that is expressed in the cell and that encodes the polypeptide.

31. A method according to claim 30, further comprising: comparing (i) the quality of the corresponding phenotype displayed by a non-human test organism or part thereof to which a synthetic construct comprising a tandem repeat of the first codon was provided; and (ii) the quality of the corresponding phenotype displayed by a non-human test organism or part thereof to which a synthetic construct comprising a tandem repeat of a second codon was provided, wherein the second codon encodes the same amino acid as the first codon, to thereby determine the phenotypic preference of the first codon relative to the phenotypic preference of the second codon in the test organism or part.

32. A method according to claim 30, further comprising: (1) introducing the synthetic construct into a progenitor of a non-human test organism or part thereof; and (2) producing the test organism or part from the progenitor wherein the test organism or part contains the synthetic construct.

33. A method according to claim 30, further comprising: (1) introducing the synthetic construct into a progenitor of a non-human test organism or part thereof; and (2) growing the test organism or part from the progenitor; wherein the test organism or part comprises a cell containing the synthetic construct.

34. A method according to claim 30, further comprising: introducing the synthetic construct into a selected cell of the non-human test organism or part.

35. A method for determining the phenotypic preference of a first codon in a multicellular organism of interest or part thereof, the method comprising: (a) introducing a synthetic construct into an *ex vivo* part of a test organism, wherein said part of a test organism is selected from tissues or organs of the test organism, wherein said part of a test organism does not include a human embryonic stem cell or a human germ line cell, wherein the part of a test organism is selected from the group consisting of an organism of the same species as the organism of interest and an organism that is related to the organism of interest, the synthetic construct comprising a regulatory polynucleotide operably linked to a tandem repeat of the first codon fused in frame with a reporter polynucleotide that encodes a reporter protein, which produces, or which is predicted to produce, a selected phenotype or a phenotype of the same class as the selected phenotype; and (b) determining the quality of the corresponding phenotype displayed by the *ex vivo* part of a test organism, wherein the selected phenotype or the phenotype of the same class as the selected phenotype is other than a phenotype conferred upon a cell by the parent polynucleotide containing the first codon that is expressed in the cell and that encodes the polypeptide.

36. A method according to claim 35, further comprising: comparing (i) the quality of the corresponding phenotype displayed by an ex *vivo* part of a test organism to which a synthetic construct comprising a tandem repeat of the first codon was provided, wherein said part of a test organism is selected from tissues or organs of the test organism, wherein said part of a test organism does not include a human embryonic stem cell or a human germ line cell; and (ii) the quality of the corresponding phenotype displayed by an *ex vivo* part of a test organism to which a synthetic construct comprising a tandem repeat of a second codon was provided, wherein said part of a test organism is selected from tissues or organs of the test organism, wherein said part of a test organism does not include a human embryonic stem cell or a human germ line cell, wherein the second codon encodes the same amino acid as the first codon, to thereby determine the phenotypic preference of the first codon relative to the phenotypic preference of the second codon in the test organism or part.

## Patentansprüche

1. Verfahren zum Konstruieren eines synthetischen Polynukleotids, aus dem ein Polypeptid erzeugbar ist, um einem multizellulären Organismus von Interesse oder einem Teil davon einen ausgewählten Phänotyp in einer anderen Qualität als jener zu verleihen, die von einem Ursprungspolynukleotid verliehen wird, das für dasselbe Polypeptid kodiert, wobei das Verfahren Folgendes umfasst:
(a) Auswählen eines ersten Kodons des Ursprungspolynukleotids für einen Austausch durch ein synonymes Kodon, wobei das synonyme Kodon auf der Basis ausgewählt wird, dass es in einem Vergleich phänotypischer Präferenzen in nicht humanen Testorganismen oder Teilen davon eine andere phänotypische Präferenz aufweist als das erste Kodon, wobei die Teile von Testorganismen aus Geweben oder Organen der Testorganismen ausgewählt sind, wobei die Testorganismen ausgewählt sind aus der Gruppe bestehend aus Organismen derselben Spezies wie der Organismus von Interesse und Organismen, die mit dem Organismus von Interesse verwandt sind; und (b) Austauschen des ersten Kodons durch das synonyme Kodon zur Konstruktion des synthetischen Polynukleotids, wobei der ausgewählte Phänotyp anders als ein Phänotyp ist, der einer Zelle durch das Ursprungspolynukleotid verliehen wird, das das erste Kodon enthält, das in der Zelle exprimiert wird und das für das Polypeptid kodiert.

2. Verfahren nach Anspruch 1, wobei die phänotypischen Präferenzen von Kodons in den Testorganismen oder Teilen verglichen werden durch: (i) separates Einführen individueller synthetischer Konstrukte in die Testorganismen oder Teile, von welchen jedes ein regulatorisches Polynukleotid umfasst, das funktionsfähig an eine Tandem-Wiederholung eines Kodons gebunden ist, die im Leseraster mit einem Reporter-Polynukleotid fusioniert ist, das für ein Reporter-Protein kodiert, das einen entsprechenden Phänotyp erzeugt, oder von dem vorhergesagt wird, dass es diesen erzeugt, der ausgewählt ist aus der Gruppe bestehend aus dem ausgewählten Phänotyp und einem Phänotyp derselben Klasse wie der ausgewählte Phänotyp; und (ii) Vergleichen der Qualität der Phänotypen, die von den Testorganismen oder Teilen davon präsentiert werden, um die relativen phänotypischen Präferenzen der Kodons zu bestimmen.

3. Verfahren zum Konstruieren eines synthetischen Polynukleotids, aus dem ein Polypeptid erzeugbar ist, um einem multizellulären Organismus von Interesse oder einem Teil davon einen ausgewählten Phänotyp in einer anderen Qualität als jener zu verleihen, die von einem Ursprungspolynukleotid verliehen wird, das für dasselbe Polypeptid kodiert, wobei das Verfahren Folgendes umfasst:
(a) Auswählen eines ersten Kodons des Ursprungspolynukleotids für einen Austausch durch ein synonymes Kodon, wobei das synonyme Kodon auf der Basis ausgewählt wird, dass es in einem Vergleich phänotypischer Präferenzen in ex vivo Teilen von Testorganismen eine andere phänotypische Präferenz aufweist als das erste Kodon, wobei die Teile von Testorganismen aus Geweben oder Organen der Testorganismen ausgewählt sind, wobei die Teile von Testorganismen keine humane embryonale Stammzelle oder humane Keimlinienzelle enthalten, wobei die Teile von Testorganismen ausgewählt sind aus der Gruppe bestehend aus Organismen derselben Spezies wie der Organismus von Interesse und Organismen, die mit dem Organismus von Interesse verwandt sind; und (b) Austauschen des ersten Kodons durch das synonyme Kodon zur Konstruktion des synthetischen Polynukleotids, wobei der ausgewählte Phänotyp anders als ein Phänotyp ist, der einer Zelle durch das Ursprungspolynukleotid verliehen wird, das das erste Kodon enthält, das in der Zelle exprimiert wird und das für das Polypeptid kodiert.

4. Verfahren nach Anspruch 3, wobei die phänotypischen Präferenzen von Kodons in den Teilen von Testorganismen verglichen werden durch: (i) separates Einführen individueller synthetischer Konstrukte in die ex vivo Teile von Testorganismen, von welchen jedes ein regulatorisches Polynukleotid umfasst, das funktionsfähig an eine Tandem-Wiederholung eines Kodons gebunden ist, die im Leseraster mit einem Reporter-Polynukleotid fusioniert ist, das für ein Reporter-Protein kodiert, das einen entsprechenden Phänotyp erzeugt, oder von dem vorhergesagt wird, dass es diesen erzeugt, der ausgewählt ist aus der Gruppe bestehend aus dem ausgewählten Phänotyp und einem Phänotyp derselben Klasse wie der ausgewählte Phänotyp; und (ii) Vergleichen der Qualität der Phänotypen, die von den ex vivo Teilen von Testorganismen präsentiert werden, um die relativen phänotypischen Präferenzen der Kodons zu bestimmen.

5. Verfahren nach Anspruch 2 oder 4, wobei das Reporter-Protein den ausgewählten Phänotyp erzeugt.

6. Verfahren nach Anspruch 2 oder 4, wobei der Reporter den ausgewählten Phänotyp nicht erzeugt, sondern dieselbe Klasse von Phänotyp wie den ausgewählten Phänotyp erzeugt.

7. Verfahren nach Anspruch 5 oder 6, wobei das Reporter-Protein ausgewählt ist aus Antigenen, die von pathogenen Organismen, Krebs-Antigenen, Auto-Antigenen, Transplantations-Antigenen, Wachstumsfaktoren, Hormonen und Toxinen abgeleitet sind.

8. Verfahren nach einem der Ansprüche 1 bis 7, wobei der Phänotyp ausgewählt ist aus Immunität, Antigentoleranz, Angiogenese, Anti-Angiogenese, Verbesserung klinischer Symptome, verringertem oder erhöhtem Zelltod, verringerter oder erhöhter Zelldifferenzierung, verringerter oder erhöhter Zellvermehrung, Tumor- oder Krebsrückbildung, Wachstum und Reparatur von Gewebe oder Organ, verminderter Fibrose, Hemmung oder Umkehr einer Zellseneszenz, verringerter oder erhöhter Zellmigration, Differenzialexpression von Protein zwischen verschiedenen Zellen oder Geweben eines Organismus oder Teiles davon, Erholung von einem Trauma, Erholung von Verbrennungen, Antibiotikaresistenz oder -empfindlichkeit, Herbizidtoleranz oder -empfindlichkeit Stärke-Biosynthese oder -Modifizierung, Fettsäurebiosynthese, Krankheitsresistenz oder -toleranz, Schädlingsresistenz oder -toleranz, einschließlich Insektenresistenz oder - toleranz, Virusresistenz oder -toleranz, Pilzresistenz oder -toleranz, einem metabolischen Merkmal, einschließlich Sucrosemetabolismus, Frostresistenz oder - toleranz, Stresstoleranz und verbessertem Nährmittelgehalt oder erhöhten Erträgen.

9. Verfahren nach einem der Ansprüche 1 bis 7, wobei der Phänotyp eine Immunantwort ist.

10. Verfahren nach Anspruch 9, wobei die Immunantwort eine humorale Immunantwort ist.

11. Verfahren nach Anspruch 9, wobei die Immunantwort eine zellvermittelte Immunantwort ist.

12. Verfahren nach Anspruch 9, wobei die Immunantwort eine durch angeborene Immunität vermittelte Antwort ist.

13. Verfahren nach Anspruch 2 oder Anspruch 4, wobei die synthetischen Konstrukte in die nicht humanen Testorganismen unter Verwendung desselben oder ähnlichen Einführungsmodus eingeführt werden.

14. Verfahren nach Anspruch 2 oder Anspruch 4, wobei die synthetischen Konstrukte in die nicht humanen Testorganismen an derselben oder entsprechenden Stelle eingeführt werden.

15. Verfahren nach Anspruch 1, wobei der Organismus von Interesse ein nicht humanes Säugetier ist und die synthetischen Konstrukte über eine orale, intravenöse, intramuskuläre, intranasale, buccale, subkutane, transdermale, buccale oder sublinguale Route eingeführt werden.

16. Verfahren nach Anspruch 1, wobei die synthetischen Konstrukte in eine oder mehrere Zell- oder Gewebearten des Organismus von Interesse eingeführt werden.

17. Verfahren nach Anspruch 16, wobei die synthetischen Konstrukte in Zellen eingeführt werden, die ausgewählt sind aus Muskelzellen, Hautzellen, Gehirnzellen, Lungenzellen, Nierenzellen, Pankreaszellen, Zellen eines Fortpflanzungsorgans, Herzzellen, Gefäßzellen, Leberzellen, Augenzellen, Blumenzellen, meristematischen Zellen, Wurzelzellen und Blattzellen.

18. Verfahren nach Anspruch 3, wobei die synthetischen Konstrukte in eine oder mehrere ex vivo Zell- oder Gewebearten des Organismus von Interesse eingeführt werden, wobei die Zell- oder Gewebearten keine humane embryonale Stammzelle oder humane Keimlinienzelle enthalten.

19. Verfahren nach Anspruch 18, wobei die Zellen, in die die synthetischen Konstrukte eingeführt werden, ausgewählt sind aus Muskelzellen, Hautzellen, Gehirnzellen, Lungenzellen, Nierenzellen, Pankreaszellen, Zellen eines Fortpflanzungsorgans, Herzzellen, Gefäßzellen, Leberzellen, Augenzellen, Blumenzellen, meristematischen Zellen, Wurzelzellen und Blattzellen.

20. Verfahren nach Anspruch 2 oder 4, wobei die Tandemwiederholung jedes der synthetischen Konstrukte mindestens drei Kopien des entsprechenden Kodons umfasst.

21. Verfahren nach einem der Ansprüche 1 bis 4, wobei das synonyme Kodon so ausgewählt ist, dass es eine höhere phänotypische Präferenz aufweist als das erste Kodon.

22. Verfahren nach Anspruch 21, wobei das synonyme Kodon ausgewählt wird, wenn die Qualität des Phänotyps, der durch das synthetische Konstrukt verliehen wird, das eine Tandemwiederholung des synonymen Kodons umfasst, mindestens etwa 5% höher ist als die Qualität des Phänotyps, der durch das synthetische Konstrukt verliehen wird, das eine Tandemwiederholung des ersten Kodons umfasst.

23. Verfahren nach einem der Ansprüche 1 bis 4, wobei das synonyme Kodon so ausgewählt ist, dass es eine geringere phänotypische Präferenz aufweist als das erste Kodon.

24. Verfahren nach Anspruch 23, wobei das synonyme Kodon ausgewählt wird, wenn die Qualität des Phänotyps, der durch das synthetische Konstrukt verliehen wird, das eine Tandemwiederholung des synonymen Kodons umfasst, mindestens etwa 5% niedriger ist als die Qualität des Phänotyps, der durch das synthetische Konstrukt verliehen wird, das eine Tandemwiederholung des ersten Kodons umfasst.

25. Verfahren nach einem der Ansprüche 1 bis 24, wobei der multizelluläre Organismus ein Tier ist.

26. Verfahren nach einem der Ansprüche 1 bis 24, wobei der multizelluläre Organismus ein Säugetier ist.

27. Verfahren nach einem der Ansprüche 1 bis 24, wobei der multizelluläre Organismus eine Pflanze ist.

28. Verfahren nach Anspruch 2, wobei die synthetischen Konstrukte in Vorläufer der nicht humanen Testorganismen oder Teile eingeführt werden und die Vorläufer für eine Zeitperiode und unter Bedingungen wachsen gelassen oder gezüchtet werden, die ausreichend sind, um die Testorganismen oder Teile zu erzeugen, wodurch die synthetischen Konstrukte in einer oder mehreren Zellarten dieser Organismen oder Teile enthalten sind.

29. Verfahren nach Anspruch 28, wobei die Vorläuferzellen aus Stammzellen, pluripotenten Zellen, meristematischen Zellen und embryonalem Kallus ausgewählt sind.

30. Verfahren zum Bestimmen der phänotypischen Präferenz eines ersten Kodons in einem multizellulären Organismus von Interesse oder Teil davon, wobei das Verfahren Folgendes umfasst: (a) Einführen eines synthetischen Konstrukts in einen nicht humanen Testorganismus oder Teil davon, wobei der Teil eines Testorganismus ausgewählt ist aus Geweben oder Organen des Testorganismus, wobei der Testorganismus ausgewählt ist aus der Gruppe bestehend aus einem Organismus derselben Spezies wie der Organismus von Interesse und einem Organismus, der mit dem Organismus von Interesse verwandt ist, wobei das synthetische Konstrukt ein regulatorisches Polynukleotid umfasst, das funktionsfähig an eine Tandem-Wiederholung des ersten Kodons gebunden ist, die im Leseraster mit einem Reporter-Polynukleotid fusioniert ist, das für ein Reporter-Protein kodiert, das einen ausgewählten Phänotyp oder einen Phänotyp derselben Klasse wie der ausgewählte Phänotyp erzeugt, oder von dem vorhergesagt wird, dass es diesen erzeugt; und (b) Bestimmen der Qualität des entsprechenden Phänotyps, der von dem nicht humanen Testorganismus oder Teil präsentiert wird, wobei der ausgewählte Phänotyp oder der Phänotyp derselben Klasse wie der ausgewählte Phänotyp anders ist als ein Phänotyp, der einer Zelle von dem Ursprungspolynukleotid verliehen wird, das das erste Kodon enthält, das in der Zelle exprimiert wird und das für das Polypeptid kodiert.

31. Verfahren nach Anspruch 30, des Weiteren umfassend:
Vergleichen (i) der Qualität des entsprechenden Phänotyps, der von einem nicht humanen Testorganismus oder Teil davon präsentiert wird, dem ein synthetisches Konstrukt bereitgestellt wurde, das eine Tandemwiederholung des ersten Kodons umfasst; und (ii) der Qualität des entsprechenden Phänotyps, der von einem nicht humanen Testorganismus oder Teil davon präsentiert wird, dem ein synthetisches Konstrukt bereitgestellt wurde, das eine Tandemwiederholung eines zweiten Kodons umfasst, wobei das zweite Kodon für dieselbe Aminosäure kodiert wie das erste Kodon, um **dadurch** die phänotypische Präferenz des ersten Kodons relativ zu der phänotypischen Präferenz des zweiten Kodons in dem Testorganismus oder Teil zu bestimmen.

32. Verfahren nach Anspruch 30, des Weiteren umfassend: (1) Einführen des synthetischen Konstrukts in einen Vorläufer eines nicht humanen Testorganismus oder Teiles davon; und (2) Erzeugen des Testorganismus oder Teiles davon aus dem Vorläufer, wobei der Testorganismus oder Teil das synthetische Konstrukt enthält.

33. Verfahren nach Anspruch 30, des Weiteren umfassend: (1) Einführen des synthetischen Konstrukts in einen Vorläufer eines nicht humanen Testorganismus oder Teiles davon; und (2) Wachsenlassen des Testorganismus oder Teiles davon aus dem Vorläufer, wobei der Testorganismus oder Teil eine Zelle umfasst, die das synthetische Konstrukt enthält.

34. Verfahren nach Anspruch 30, des Weiteren umfassend:
Einführen des synthetischen Konstrukts in eine ausgewählte Zelle des nicht humanen Testorganismus oder Teiles.

35. Verfahren zum Bestimmen der phänotypischen Präferenz eines ersten Kodons in einem multizellulären Organismus von Interesse oder Teil davon, wobei das Verfahren Folgendes umfasst: (a) Einführen eines synthetischen Konstrukts in einen ex vivo Teil eines Testorganismus, wobei der Teil eines Testorganismus ausgewählt ist aus Geweben oder Organen des Testorganismus, wobei der Teil eines Testorganismus keine humane embryonale Stammzelle oder humane Keimlinienzelle enthält, wobei der Teil eines Testorganismus ausgewählt ist aus der Gruppe bestehend aus einem Organismus derselben Spezies wie der Organismus von Interesse und einem Organismus, der mit dem Organismus von Interesse verwandt ist, wobei das synthetische Konstrukt ein regulatorisches Polynukleotid umfasst, das funktionsfähig an eine Tandem-Wiederholung des ersten Kodons gebunden ist, die im Leseraster mit einem Reporter-Polynukleotid fusioniert ist, das für ein Reporter-Protein kodiert, das einen ausgewählten Phänotyp oder einen Phänotyp derselbe Klasse wie der ausgewählt Phänotyp erzeugt, oder von dem vorhergesagt wird, dass es diesen erzeugt; und (b) Bestimmen der Qualität des entsprechenden Phänotyps, der von dem ex vivo Teil eines Testorganismus präsentiert wird, wobei der ausgewählte Phänotyp oder der Phänotyp derselben Klasse wie der ausgewählte Phänotyp anders ist als ein Phänotyp, der einer Zelle von dem Ursprungspolynukleotid verliehen wird, das das erste Kodon enthält, das in der Zelle exprimiert wird, und das für das Polypeptid kodiert.

36. Verfahren nach Anspruch 35, des Weiteren umfassend:
Vergleichen (i) der Qualität des entsprechenden Phänotyps, der von einem ex vivo Teil eines Testorganismus präsentiert wird, dem ein synthetisches Konstrukt bereitgestellt wurde, das eine Tandemwiederholung des ersten Kodons umfasst, wobei der Teil eines Testorganismus ausgewählt ist aus Geweben oder Organen des Testorganismus, wobei der Teil eines Testorganismus keine humane embryonale Stammzelle oder humane Keimlinienzelle enthält; und (ii) der Qualität des entsprechenden Phänotyps, der von einem ex vivo Teil eines Testorganismus präsentiert wird, dem ein synthetisches Konstrukt bereitgestellt wurde, das eine Tandemwiederholung eines zweiten Kodons umfasst, wobei der Teil eines Testorganismus ausgewählt ist aus Geweben oder Organen des Testorganismus, wobei der Teil eines Testorganismus keine humane embryonale Stammzelle oder humane Keimlinienzelle enthält, wobei das zweite Kodon für dieselbe Aminosäure kodiert wie das erste Kodon, um **dadurch** die phänotypische Präferenz des ersten Kodons relativ zu der phänotypischen Präferenz des zweiten Kodons in dem Testorganismus oder Teil zu bestimmen.

## Revendications

1. Procédé pour élaborer un polynucléotide synthétique à partir duquel un polypeptide est productible pour conférer un phénotype choisi à un organisme multicellulaire d'intérêt ou une partie de celui-ci dans une qualité différente de celle conférée par un polynucléotide parent qui code le même polypeptide, le procédé comprenant : (a) le choix d'un premier codon du polynucléotide parent pour le remplacer par un codon synonyme, dans lequel le codon synonyme est choisi parce qu'il présente une préférence phénotypique différente de celle du premier codon dans une comparaison de préférences phénotypiques dans des organismes d'essai non humains ou des parties de ceux-ci, dans lesquelles les parties d'organismes d'essai sont choisies parmi des tissus ou des organes des organismes d'essai, dans lesquels les organismes d'essai étant choisis dans le groupe constitué par les organismes de la même espèce que l'organisme d'intérêt et les organismes qui sont apparentés à l'organisme d'intérêt ; et (b) le remplacement du premier codon par le codon synonyme pour élaborer le polynucléotide synthétique, dans lequel le phénotype choisi est autre qu'un phénotype conféré à une cellule par le polynucléotide parent contenant le premier codon qui est exprimé dans la cellule et qui code le polypeptide.

2. Procédé selon la revendication 1, dans lequel les préférences phénotypiques des codons dans les organismes d'essai ou des parties de ceux-ci sont comparées : (i) en introduisant séparément dans les organismes d'essai ou des parties de ceux-ci, des constructions synthétiques individuelles, dont chacune comprend un polynucléotide régulateur lié de façon opérationnelle à une séquence répétée en tandem d'un codon fusionnée dans le cadre avec un polynucléotide rapporteur qui code une protéine rapporteuse, qui produit, ou qui devrait produire, un phénotype correspondant choisi dans le groupe constitué par le phénotype choisi et un phénotype de la même classe que le phénotype choisi ; et (ii) en comparant la qualité des phénotypes présentés par les organismes d'essai ou des parties de ceux-ci pour déterminer les préférences phénotypiques relatives des codons.

3. Procédé pour élaborer un polynucléotide synthétique à partir duquel un polypeptide est productible pour conférer un phénotype choisi à un organisme multicellulaire d'intérêt ou une partie de celui-ci dans une qualité différente de celle conférée par un polynucléotide parent qui code le même polypeptide, le procédé comprenant : (a) le choix d'un premier codon du polynucléotide parent pour le remplacer par un codon synonyme, dans lequel le codon synonyme étant choisi parce qu'il présente une préférence phénotypique différente de celle du premier codon dans une comparaison de préférences phénotypiques dans des parties ex *vivo* d'organismes d'essai, dans lesquelles lesdites parties d'organismes d'essai sont choisies parmi des tissus ou des organes des organismes d'essai, dans lesquelles lesdites parties d'organismes d'essai n'inclue pas une cellule souche embryonnaire humaine ou une cellule de lignée germinale humaine, dans lesquelles lesdites parties d'organismes d'essai sont choisies dans le groupe constitué par les organismes de la même espèce que l'organisme d'intérêt et les organismes qui sont apparentés à l'organisme d'intérêt ; et (b) le remplacement du premier codon par le codon synonyme pour élaborer le polynucléotide synthétique, dans lequel le phénotype choisi est autre qu'un phénotype conféré à une cellule par le polynucléotide parent contenant le premier codon qui est exprimé dans la cellule et qui code le polypeptide.

4. Procédé selon la revendication 3, dans lequel les préférences phénotypiques des codons dans les parties d'organismes d'essai sont comparées : (i) en introduisant, séparément dans les parties ex *vivo* d'organismes d'essai, des constructions synthétiques individuelles, dont chacun comprend un polynucléotide régulateur lié de façon opérationnelle à une séquence répétée en tandem d'un codon fusionnée dans le cadre avec un polynucléotide rapporteur qui code une protéine rapporteuse, qui produit, ou qui devrait produire, un phénotype correspondant choisi dans le groupe constitué par le phénotype choisi et un phénotype de la même classe que le phénotype choisi ; et (ii) en comparant la qualité des phénotypes présentés par les parties ex *vivo* d'organismes d'essai pour déterminer les préférences phénotypiques relatives des codons.

5. Procédé selon la revendication 2 ou 4, dans lequel la protéine rapporteuse produit le phénotype choisi.

6. Procédé selon la revendication 2 ou 4, dans lequel le rapporteur ne produit pas le phénotype choisi mais produit la même classe de phénotype que le phénotype choisi.

7. Procédé selon la revendication 5 ou la revendication 6, dans lequel la protéine rapporteuse est choisie parmi les antigènes dérivés d'organismes pathogènes, les antigènes cancéreux, les auto-antigènes, les antigènes de transplantation, les facteurs de croissance, les hormones et les toxines.

8. Procédé selon l'une quelconque des revendications 1 à 7, dans lequel le phénotype est choisi parmi une immunité, une tolérance antigénique, une angiogenèse, une anti-angiogenèse, une amélioration de symptômes cliniques, une réduction ou une augmentation de la mort cellulaire, une réduction ou une augmentation de la différenciation cellulaire, une réduction ou une augmentation de la prolifération cellulaire, une régression tumorale ou cancéreuse, une croissance et une réparation d'un tissu ou d'un organe, une fibrose diminuée, une inhibition ou une inversion de sénescence cellulaire, une augmentation ou une diminution de la migration cellulaire, une expression différentielle de protéine entre des cellules ou des tissus différents d'un organisme ou d'une partie de celui-ci, un rétablissement d'un traumatisme, un rétablissement de brûlures, une résistance ou une sensibilité à un antibiotique, une tolérance ou une sensibilité à un herbicide, une biosynthèse ou une modification d'amidon, une biosynthèse d'acide gras, une résistance ou une tolérance à une maladie, une résistance ou une tolérance à un nuisible incluant une résistance ou une tolérance à un insecte, une résistance ou une tolérance virale, une résistance ou une tolérance fongique, un trait métabolique incluant le métabolisme du saccharose, une résistance ou une tolérance au gel, une tolérance au stress, et un contenu alimentaire amélioré ou des rendements accrus.

9. Procédé selon l'une quelconque des revendications 1 à 7, dans lequel le phénotype est une réponse immunitaire.

10. Procédé selon la revendication 9, dans lequel la réponse immunitaire est une réponse immunitaire humorale.

11. Procédé selon la revendication 9, dans lequel la réponse immunitaire est une réponse immunitaire médiée par une cellule.

12. Procédé selon la revendication 9, dans lequel la réponse immunitaire est une réponse médiée par l'immunité innée.

13. Procédé selon la revendication 2 ou la revendication 4, dans lequel les constructions synthétiques sont introduites dans les organismes d'essai non humains en utilisant un mode d'introduction identique ou semblable.

14. Procédé selon la revendication 2 ou la revendication 4, dans lequel les constructions synthétiques sont introduites dans les organismes d'essai non humains au même site ou à un site correspondant.

15. Procédé selon la revendication 1, dans lequel l'organisme d'intérêt est un mammifère non humain et les constructions synthétiques sont introduites par la voie orale, intraveineuse, intramusculaire, intranasale, buccale, sous-cutanée, transdermique, buccale ou sublinguale.

16. Procédé selon la revendication 1, dans lequel les constructions synthétiques sont introduites dans une ou plusieurs sortes de cellules ou de tissus de l'organisme d'intérêt.

17. Procédé selon la revendication 16, dans lequel les constructions synthétiques sont introduites dans des cellules choisies parmi les cellules musculaires, les cellules cutanées, les cellules cérébrales, les cellules pulmonaires, les cellules rénales, les cellules pancréatiques, les cellules d'un organe reproducteur, les cellules cardiaques, les cellules vasculaires, les cellules hépatiques, les cellules oculaires, les cellules de fleurs, les cellules méristématiques, les cellules de racines et les cellules de feuilles.

18. Procédé selon la revendication 3, dans lequel les constructions synthétiques sont introduites dans une ou plusieurs sortes de cellules ou de tissus ex *vivo* de l'organisme d'intérêt, dans lesquelles lesdites sortes de cellules ou de tissus n'incluent pas une cellule souche embryonnaire humaine ou une cellule de lignée germinale humaine.

19. Procédé selon la revendication 18, dans lequel les cellules dans lesquelles les constructions synthétiques sont introduites sont choisies parmi les cellules musculaires, les cellules cutanées, les cellules cérébrales, les cellules pulmonaires, les cellules rénales, les cellules pancréatiques, les cellules d'un organe reproducteur, les cellules cardiaques, les cellules vasculaires, les cellules hépatiques, les cellules oculaires, les cellules de fleurs, les cellules méristématiques, les cellules de racines et les cellules de feuilles.

20. Procédé selon la revendication 2 ou 4, dans lequel la séquence répétée en tandem de chacune des constructions synthétiques comprend au moins trois copies du codon correspondant.

21. Procédé selon l'une quelconque des revendications 1 à 4, dans lequel le codon synonyme est choisi de sorte qu'il a une préférence phénotypique plus élevée que le premier codon.

22. Procédé selon la revendication 21, dans lequel le codon synonyme est choisi lorsque la qualité du phénotype conféré par la construction synthétique comprenant une séquence répétée en tandem du codon synonyme est au moins environ 5 % plus élevée que la qualité du phénotype conféré par la construction synthétique comprenant une séquence répétée en tandem du premier codon.

23. Procédé selon l'une quelconque des revendications 1 à 4, dans lequel le codon synonyme est choisi de sorte qu'il a une préférence phénotypique plus basse que le premier codon.

24. Procédé selon la revendication 23, dans lequel le codon synonyme est choisi lorsque la qualité du phénotype conféré par le produit d'assemblage synthétique comprenant des séquences répétées en tandem du codon synonyme est au moins environ 5 % plus basse que la qualité du phénotype conféré par le produit d'assemblage synthétique comprenant des séquences répétées en tandem du premier codon.

25. Procédé selon l'une quelconque des revendications 1 à 24, dans lequel l'organisme multicellulaire est un animal.

26. Procédé selon l'une quelconque des revendications 1 à 24, dans lequel l'organisme multicellulaire est un mammifère.

27. Procédé selon l'une quelconque des revendications 1 à 24, dans lequel l'organisme multicellulaire est une plante.

28. Procédé selon la revendication 2, dans lequel les constructions synthétiques sont introduites dans des progéniteurs des organismes d'essai non humains ou des parties de ceux-ci, et les progéniteurs sont développés ou cultivés pendant une durée et dans des conditions suffisantes pour produire les organismes d'essai ou des parties de ceux-ci, de sorte que les constructions synthétiques sont contenus dans une ou plusieurs sortes cellulaires de ces organismes ou de parties de ceux-ci.

29. Procédé selon la revendication 28, dans lequel les cellules progénitrices sont choisies parmi les cellules souches, les cellules pluripotentielles, les cellules méristématiques et un cal embryonnaire.

30. Procédé pour déterminer la préférence phénotypique d'un premier codon dans un organisme multicellulaire d'intérêt ou une partie de celui-ci, le procédé comprenant : (a) l'introduction d'une construction synthétique dans un organisme d'essai non humain ou une partie de celui-ci, dans laquelle ladite partie d'un organisme d'essai est choisie parmi des tissus ou des organes de l'organisme d'essai, dans laquelle l'organisme d'essai est choisi dans le groupe constitué par un organisme de la même espèce que l'organisme d'intérêt et un organisme qui est apparenté à l'organisme d'intérêt, la construction synthétique comprenant un polynucléotide régulateur lié de façon opérationnelle à une séquence répétée en tandem du premier codon fusionnée dans le cadre avec un polynucléotide rapporteur qui code une protéine rapporteuse, qui produit, ou qui devrait produire, un phénotype choisi ou un phénotype de la même classe que le phénotype choisi ; et (b) la détermination de la qualité du phénotype correspondant présenté par l'organisme d'essai non humain ou une partie de celui-ci, dans laquelle le phénotype choisi ou le phénotype de la même classe que le phénotype choisi est autre qu'un phénotype conféré à une cellule par le polynucléotide parent contenant le premier codon qui est exprimé dans la cellule et qui code le polypeptide.

31. Procédé selon la revendication 30, comprenant en outre : la comparaison (i) de la qualité du phénotype correspondant présenté par un organisme d'essai non humain ou une partie de celui-ci auquel une construction synthétique comprenant une séquence répétée en tandem du premier codon a été fourni ; et (ii) de la qualité du phénotype correspondant présenté par un organisme d'essai non humain ou une partie de celui-ci auquel une construction synthétique comprenant une séquence répétée en tandem d'un second codon a été fourni, dans laquelle le second codon codant le même acide aminé que le premier codon, pour déterminer ainsi la préférence phénotypique du premier codon par rapport à la préférence phénotypique du second codon dans l'organisme d'essai ou la partie de celui-ci.

32. Procédé selon la revendication 30, comprenant en outre : (i) l'introduction de la construction synthétique dans un progéniteur d'un organisme d'essai non humain ou une partie de celui-ci ; et (2) la production de l'organisme d'essai ou d'une partie de celui-ci à partir du progéniteur, dans laquelle l'organisme d'essai ou la partie de celui-ci contient la construction synthétique.

33. Procédé selon la revendication 30, comprenant en outre : (1) l'introduction de la construction synthétique dans un progéniteur d'un organisme d'essai non humain ou une partie de celui-ci ; et (2) le développement de l'organisme d'essai ou d'une partie de celui-ci à partir du progéniteur ; dans lequel l'organisme d'essai ou la partie de celui-ci comprend une cellule contenant la construction synthétique.

34. Procédé selon la revendication 30, comprenant en outre : l'introduction de la construction synthétique dans une cellule choisie de l'organisme d'essai non humain ou une partie de celui-ci.

35. Procédé pour déterminer la préférence phénotypique d'un premier codon dans un organisme multicellulaire d'intérêt ou une partie de celui-ci, le procédé comprenant : (a) l'introduction d'une construction synthétique dans une partie ex *vivo* d'un organisme d'essai, dans laquelle la partie d'un organisme d'essai est choisie parmi des tissus ou des organes de l'organisme d'essai, dans laquelle ladite partie d'un organisme d'essai n'inclue pas une cellule souche embryonnaire humaine ou une cellule de lignée germinale humaine, dans laquelle la partie d'un organisme d'essai est choisie dans le groupe constitué par un organisme de la même espèce que l'organisme d'intérêt et un organisme qui est apparenté à l'organisme d'intérêt, la construction synthétique comprenant un polynucléotide régulateur lié de façon opérationnelle à une séquence répétée en tandem du premier codon fusionnée dans le cadre avec un polynucléotide rapporteur qui code une protéine rapporteuse, qui produit, ou qui devrait produire, un phénotype choisi ou un phénotype de la même classe que le phénotype choisi ; et (b) la détermination de la qualité du phénotype correspondant présenté par la partie ex *vivo* d'un organisme d'essai, dans lequel le phénotype choisi ou le phénotype de la même classe que le phénotype choisi est autre qu'un phénotype conféré à une cellule par le polynucléotide parent contenant le premier codon qui est exprimé dans la cellule et qui code le polypeptide.

36. Procédé selon la revendication 35, comprenant en outre : la comparaison (i) de la qualité du phénotype correspondant présenté par une partie ex *vivo* d'un organisme d'essai à laquelle une construction synthétique comprenant une séquence répétée en tandem du premier codon a été fournie, dans laquelle ladite partie d'un organisme d'essai est choisie parmi des tissus ou des organes de l'organisme d'essai, dans laquelle ladite partie d'un organisme d'essai n'inclue pas une cellule souche embryonnaire humaine ou une cellule de lignée germinale humaine ; et (ii) de la qualité du phénotype correspondant présenté par une partie ex *vivo* d'un organisme d'essai à laquelle une construction synthétique comprenant une séquence répétée en tandem d'un second codon a été fourni, dans laquelle ladite partie d'un organisme d'essai est choisie parmi des tissus ou des organes de l'organisme d'essai, dans laquelle ladite partie d'un organisme d'essai n'inclue pas une cellule souche embryonnaire humaine ou une cellule de lignée germinale humaine, dans lequel le second codon codant le même acide aminé que le premier codon, pour déterminer ainsi la préférence phénotypique du premier codon par rapport à la préférence phénotypique du second codon dans l'organisme d'essai ou une partie de celui-ci.
